# EUROPEAN PATENT APPLICATION

(11) **EP 4 353 303 A2**
(43) Date of publication of application: **17.04.2024**
(21) Application number: 23202924.9
(22) Date of filing: 11.10.2023
(51) Int. Cl.: A61N 1/36, A61N 1/372, G16H 20/00, G16H 40/00

(54) **INCONTINENCE THERAPY SCHEDULING**

(30) Priority: 12.10.2022 US 202263379176 P; 12.10.2022 US 202263379181 P; 22.09.2023 US 202318472344
(71) Applicant: Medtronic, Inc., Minneapolis, MN 55432 (US)
(72) Inventor: Paralikar, Kunal J., Minneapolis (US); Erler, James E., Minneapolis (US); Karumuri, Saketh, Minneapolis (US); Thimmesch-Gill, Zane K., Minneapolis (US); Singer, Nicholas R., Minneapolis (US); Pratt, Joseph N., Minneapolis (US); Skarie, Laura A., Minneapolis (US); Klotsche, Emily G., Mounds View (US)
(74) Representative: Zimmermann & Partner Patentanwälte mbB

(57) **Abstract**

In some examples, a system includes processing circuitry configured to receive a first set of information, the first set of information comprising information of actual therapy delivered to a patient over a plurality of instances of therapy delivery. The processing circuitry may determine, based upon the first set of information, a therapy usage pattern. The processing circuitry may determine a modification to a programmed therapy schedule based on the therapy usage pattern. The processing circuitry may generate for output the modification to the programmed therapy schedule.

## Description

This application claims the benefit of U.S. Provisional Application No. 63/379,176, filed October 12, 2022, and entitled, "INCONTINENCE THERAPY SCHEDULING," and the benefit U.S. Provisional Application No. 63/379,181, filed October 12, 2022, and entitled, "INCONTINENCE THERAPY SCHEDULING," the entire contents of each of which is incorporated herein by reference.

### TECHNICAL FIELD

The disclosure relates to medical devices and, more particularly, to medical devices configured to deliver electrical stimulation therapy.

### BACKGROUND

Disease, age, and injury may impair physiological functions of a patient. In some situations, the physiological functions are completely impaired. In other examples, the physiological function may operate sufficiently at some times or under some conditions and operate inadequately at other times or under other conditions. Some examples of impaired physiological functions include overactive bladder, non-obstructive urinary retention, fecal incontinence, constipation, neurogenic bladder or bowel function, pelvic pain, and sexual dysfunction. In one example, bladder dysfunction, such as overactive bladder, urgency, or urinary incontinence, is a problem that may afflict people of all ages, genders, and races. Various muscles, nerves, organs, and conduits within the pelvic floor cooperate to collect, store and release urine. A variety of disorders may compromise urinary tract performance, and contribute to an overactive bladder, urgency, or urinary incontinence that interferes with normal physiological function. Many of the disorders may be associated with aging, injury or illness.

### SUMMARY

The various example techniques described herein may personalize and optimize the care for each patient based on his or her disease burden, as well as reduce the practice burden to clinics. The example personalized and optimized care techniques described in this disclosure may be used to treat OAB (overactive bladder), urinary retention, fecal incontinence, as well as neurogenic function, pelvic pain, and sexual function. However, the example techniques described in this disclosure should not be considered limited to the example conditions, and may be used for other medical conditions as well.

The example systems techniques described herein offer a clinical workflow that steps a user through therapy with various checkpoints to either increase or decrease therapy regimen based on patient outcomes or patient goals while permitting reduction in unnecessary interaction with clinicians. Example systems may also allow for communication between devices of different programmed formats, such as different time formats.

In some examples, a system includes processing circuitry configured to receive a first set of information, the first set of information comprising information of actual therapy delivered to a patient over a plurality of instances of therapy delivery. The processing circuitry may determine based upon the first set of information, a therapy usage pattern. The processing circuitry may determine a modification to a programmed therapy schedule based on the therapy usage pattern. The processing circuitry may generate for output the modification to the programmed therapy schedule.

In some examples, a method includes receiving, via processing circuitry, a first set of information, the first set of information comprising information of actual therapy delivered to a patient over a plurality of instances of therapy delivery. The method may include determining, via processing circuitry, based upon the first set of information, a therapy usage pattern. The method may include determining, via processing circuitry, a modification to a programmed therapy schedule based on the therapy usage pattern. The method may include generating for output, via processing circuitry, the modification to the programmed therapy schedule.

In some examples, a system includes processing circuitry configured to access information of a programmed therapy schedule of a patient. The processing circuitry may calculate an epoch-based therapy schedule based on the programmed therapy schedule, wherein the epoch-based therapy schedule comprises a therapy schedule based on a count relative to a predetermined epoch date. The processing circuitry may deliver to an implantable medical device comprising an epoch-based timing system, the epoch-based therapy schedule.

In some examples, a method includes accessing, via processing circuitry, information of a programmed therapy schedule of a patient. The method may include calculating, via processing circuitry, an epoch-based therapy schedule based on the programmed therapy schedule, wherein the epoch-based therapy schedule comprises a therapy schedule based on a count relative to a predetermined epoch date. The method may include delivering, via processing circuitry, to an implantable medical device comprising an epoch-based timing system, the epoch-based therapy schedule.

In some examples, a system includes processing circuitry configured to receive a first set of information, the first set of information comprising information of actual therapy delivered to a patient over a plurality of instances of therapy delivery. The processing circuitry may determine, based upon the first set of information, a therapy usage pattern. The processing circuitry may determine a modification to a programmed therapy schedule based on the therapy usage pattern. The processing circuitry may generate for output the modification to the programmed therapy schedule.

This summary is intended to provide an overview of the subject matter described in this disclosure. It is not intended to provide an exclusive or exhaustive explanation of the systems, devices, and methods described in detail within the accompanying drawings and description below. Further details of one or more examples are set forth in the accompanying drawings and the description below. Other features, objects, and advantages will be apparent from the description and drawings, and from the statements provided below.

### BRIEF DESCRIPTION OF DRAWINGS

FIG. 1 is a conceptual diagram illustrating an example system that manages delivery of stimulation to a patient, according to one or more examples.
FIG. 2 is a conceptual diagram illustrating an example system that manages delivery of stimulation to a patient, according to one or more examples.
FIG. 3 is a block diagram of an example patient programmer, an implantable medical device, and a server according to one or more examples.
FIG. 4 is a conceptual diagram illustrating an example home screen for navigating within a user interface when first connecting to an implantable medical device (IMD).
FIG. 5 is a conceptual diagram illustrating an example setup screen for managing stimulation amplitude.
FIG. 6 is a conceptual diagram illustrating an example setup screen for IMD startup.
FIG. 7 is a conceptual diagram illustrating an example setup screen for IMD startup.
FIG. 8 is a conceptual diagram illustrating an example setup screen for managing stimulation amplitude.
FIG. 9 is a conceptual diagram illustrating an example setup screen for managing stimulation amplitude.
FIG. 10 is a conceptual diagram illustrating an example advanced setup screen for configuring stimulation.
FIG. 11 is a conceptual diagram illustrating an example setup screen for managing a stimulation schedule.
FIG. 12 is a conceptual diagram illustrating an example setup screen for managing a stimulation schedule.
FIG. 13 is a conceptual diagram illustrating an example setup screen for managing a stimulation schedule.
FIG. 14 is a conceptual diagram illustrating an example setup screen for managing a custom stimulation schedule.
FIG. 15 is a conceptual diagram illustrating an example setup screen for managing a custom stimulation schedule.
FIG. 16 is a conceptual diagram illustrating an example setup screen for managing a custom stimulation schedule.
FIG. 17 is a conceptual diagram illustrating an example review screen for managing a stimulation schedule.
FIG. 18 is a conceptual diagram illustrating an example review screen for reviewing and finishing device setup.
FIG. 19 is a conceptual diagram illustrating an example review screen for confirming device setup is complete.
FIG. 20 is a conceptual diagram illustrating an example home screen for providing a dashboard view.
FIG. 21 is a conceptual diagram illustrating an example informational screen for reviewing a patient's therapy adherence history.
FIG. 22 is a conceptual diagram illustrating an example setup screen for managing notifications of recommended therapy modifications.
FIG. 23 is a flow diagram illustrating an example technique for using a programmer to generate therapy recommendations.
FIG. 24 is a conceptual diagram illustrating a communication between a programmer and an implantable medical device via a scheduling algorithm.
FIG. 25 is a flow diagram illustrating an example technique for using scheduling algorithm to deliver a therapy schedule via an implantable medical device.
FIG. 26 is a conceptual diagram illustrating an example setup screen for managing a custom stimulation schedule.
FIG. 27 is a conceptual diagram illustrating an example screen for managing an active therapy session.
FIG. 28 is a conceptual diagram illustrating an example screen for managing an active therapy session.
FIG. 29 is a conceptual diagram illustrating an example menu screen for managing a stimulation schedule.
FIG. 30 is a conceptual diagram illustrating an example screen for managing a stimulation schedule.
FIG. 31 is a conceptual diagram illustrating an example setup screen for managing a stimulation schedule.

Like reference characters denote like elements throughout the description and figures.

### DETAILED DESCRIPTION

A patient may suffer from one or more symptoms treatable by electrical stimulation therapy. For example, a patient may suffer from incontinence (e.g., urinary and/or fecal incontinence), which may be treated by an implantable medical device (IMD) configured to output electrical stimulation therapy to stimulate one or more nerves such as tibial nerve(s), sacral nerve(s), a pudendal nerve, etc. A clinician may select or program values for various parameters relating to stimulation therapy, including a voltage or current amplitude, a pulse width, and a pulse frequency as stimulation. A clinician may also set or program a stimulation therapy schedule including the specific times/days when a patient receives therapy. A patient may alter the stimulation parameters or schedule parameters (e.g., such as, but not limited to, within set limits) before or during therapy delivery. In such cases, the therapy actually delivered to a patient may differ slightly as compared to the clinician programmed parameters or schedule. Therapy may be improved by comparing therapy actually delivered to a patient and the clinician programmed therapy parameters or schedule.

For instance, as described in more detail, processing circuitry may be configured to determine a therapy usage pattern based on instances of the actual therapy that is delivered to a patient. The term "therapy usage pattern" may refer to examples where there is a repeated, consistent way in which actual therapy is delivered (e.g., which may be different than the scheduled therapy), and may also refer to examples where the actual therapy delivery is more random. That is, determining a therapy usage pattern includes examples of determining when there is a pattern and when there is a lack of a pattern.

As described herein, various devices, systems, and techniques enable management of neuromodulation therapy. The example techniques are described with respect to tibial nerve stimulation, but can be extended to other stimulation for incontinence, such as sacral nerve stimulation, as well as for stimulation for other medical conditions.

As an example, a system may receive, store, and access information about therapy actually delivered to a patient. From the information about the therapy actually delivered, the system may determine a therapy pattern, and determine recommendations for therapy based on the therapy pattern. For instance, the system may determine modification to a programmed therapy schedule based on the therapy usage pattern.

In some examples, the system may determine the therapy pattern and/or recommendations for therapy based on comparison between scheduled therapy and actual therapy delivered. For example, systems described herein may be able to receive, store, and access information about therapy parameters or schedules as programmed by a clinician or other user. As described, the system may be able to receive, store, and access information about therapy actually delivered to a patient. The system may be able to compare information of the programmed therapy and the therapy actually delivered to the patient. Based upon the comparison, the system may be able to determine a therapy pattern. The system may determine recommendations of changes to a programmed therapy (e.g., to a clinician or other user) based on the therapy pattern. The system may output these recommendations to a user interface, allowing a clinician or other user to accept, reject, or modify the suggested therapy recommendations. In some examples, the system may automatically update the programmed therapy schedule based on the determined recommendations (e.g., such as in examples where changes to the programmed therapy schedule are within set limits).

The various features of the system described may allow for improved patient outcomes and improved system functionality. For example, the system may provide insights and recommended therapy alterations based upon one or more therapy adherence metrics. The system may prompt a user through an interface of changes to therapy stimulation parameters as well as therapy schedule. The system may also guide a clinician through setup of stimulation therapy parameters and schedules. This guidance may reduce clinician time and improve therapeutic results for the patient by increasing therapeutic stimulation efficacy and reducing side effects. The system may also employ scheduling algorithms to ensure the IMD delivers therapy according to the clinician or patient defined therapy schedules.

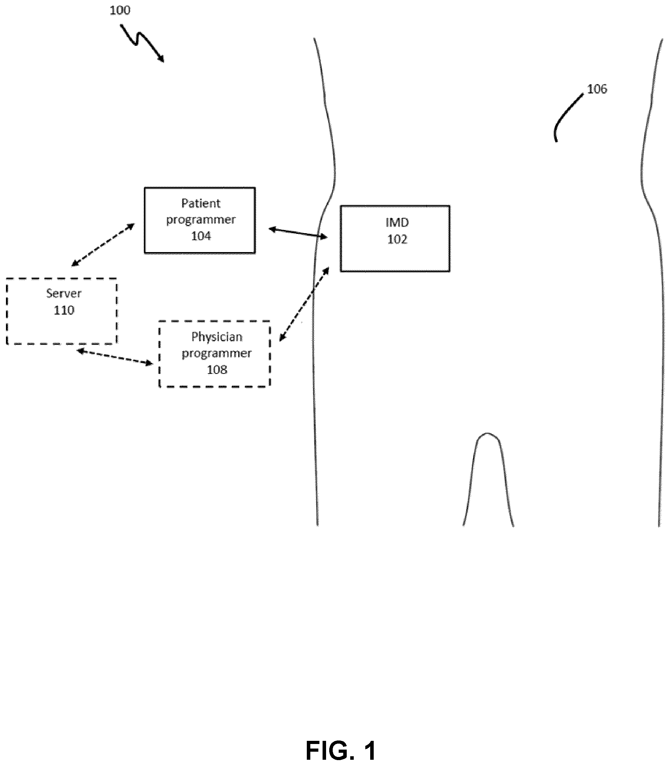

Referring to FIG. 1, a conceptual diagram illustrating an example system 100 that manages delivery of neurostimulation to a patient is depicted, according to one or more examples. System 100 generally includes an IMD 102 and a patient programmer 104 operably coupled to IMD 102.

IMD 102 can be implanted in patient 106. IMD 102 is configured to provide electrical stimulation therapy to a target tissue site corresponding to a nerve of patient 106 by generating a programmable electrical stimulation signal (e.g., in the form of electrical pulses) and delivering the electrical stimulation signal to a target tissue site. In one or more examples, IMD 102 includes a lead of one or more stimulation electrodes implanted on the nerve such that the target tissue site is the nerve itself. In one or more examples, IMD 102 is positioned adjacent to the nerve such that the electrical stimulation is delivered from IMD 102 to a target tissue site adjacent to the nerve via the stimulation electrodes.

The example techniques described in this disclosure may be used with a variety of implantable medical devices, including but not limited to nerve stimulation devices (also known as neuro stimulators or neuromodulation devices). In some examples, neuromodulation devices may be used to stimulate a variety of nerves or associated tissues for treating a variety of conditions. Electrical stimulation may be delivered for spinal cord stimulation (SCS), peripheral nerve stimulation (PNS), peripheral nerve field stimulation (PNFS), deep brain stimulation (DBS), cortical stimulation (CS), pelvic floor stimulation, sacral nerve stimulation, tibial nerve stimulation, vagus nerve stimulation, gastric stimulation, and the like.

In an example, the techniques described in this disclosure may be used as part of a system for treating pelvic health conditions including incontinence, overactive bladder, pelvic pain or other pelvic floor disorders. In some instances, the example techniques may be implemented as part of sacral nerve(s) stimulation system.

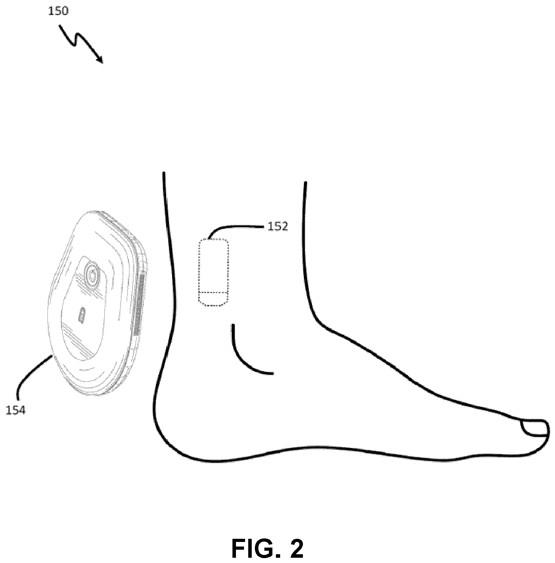

Referring to FIG. 2, in another example pertaining to treatment of pelvic health disorders, the example techniques may be implemented as part of tibial nerve(s) stimulation system 150, including an implantable tibial nerve stimulation device 152, also called IMD 152, and an external recharger 154, wherein external recharger 154 can be positioned on or proximate to skin of the patient over the location of IMD 152 to facilitate recharging. Tibial nerve stimulation system 150 may also include a wearable ankle cuff to hold external recharger 154 in position on an ankle of a patient. In some examples, tibial nerve stimulation system 150 may include a primary cell power source (e.g., non-rechargeable), and external recharger 154 may not be needed in such examples. Tibial nerve stimulation system 150 may stimulate one tibial nerve, both tibial nerves, or alternate stimulation to the tibial nerves. The example techniques are applicable to stimulation of one tibial nerve, both tibial nerves, or alternating stimulation. Tibial nerve stimulation device (i.e., IMD 152) is one example of IMD 102 of FIG. 1.

In one or more examples, IMD 102, including tibial nerve stimulation device 152, may be programmed with a therapy schedule with physician programmer 108, also called clinician programmer, and/or patient programmer 104. In this disclosure, the term "programmer" is used to generically refer to physician/clinician programmer 108 and patient programmer 104. For instance, a user (e.g., patient, physician, clinician) may interact with the programmer to select therapy parameters such as amplitude, pulse width, and frequency of therapy that is delivered to patient 106. In addition, the user may interact with the programmer to program a therapy schedule. The programmed therapy schedule may include therapy session programming such as programming for the duty cycle of the therapy (e.g., on-times and off-times), times when therapy is delivered, therapy parameters for a therapy session, etc.

Although there may be a programmed therapy schedule, patient 106 may deviate from the programmed therapy schedule. For instance, patient 106 may skip a therapy session (e.g., a time period over which therapy is be delivered as programmed), or may add therapy sessions. As additional examples of deviating from the programmed therapy schedule, patient 106 may adjust a therapy parameter in the middle of a therapy session, patient 106 may stop a therapy session midway, patient 106 may extend a therapy session, etc. In some examples, patient 106 may deviate from the programmed therapy schedule in some random ways.

In one or more examples, processing circuitry, such as processing circuitry of a programmer, processing circuitry of server 110, or a combination thereof, may determine a therapy usage pattern (which includes examples of randomness) of the actual therapy that is delivered to patient 106. The actual therapy that is delivered may be a deviation from the programmed therapy schedule, and the processing circuitry may utilize the therapy usage pattern to determine a modification to the programmed therapy schedule. For instance, if patient 106 is purposefully deviating from the programmed therapy schedule, patient 106 may find the programmed therapy schedule to be ineffective, uncomfortable, etc. With the example techniques, IMD 102 may be reprogrammed to deliver therapy that aligns with behavior patient 106 is exhibiting.

Moreover, in some examples, IMD 102 may be configured to have relatively flexible programming. That is, the programmer may allow a user to define many different parameters for defining a programmed therapy schedule, such as flexibility in defining which days to deliver, including specifying specific days, flexibility in changes to parameters, flexibility in duration, etc. Since the user has many options to define the therapy schedule, IMD 102 may utilize some form of real-world clock or real-time-based system (e.g., a clock that can specify current calendar day and time of day) to accommodate such programming. However, inclusion of a real-world clock may utilize excessive memory in IMD 102 where memory may otherwise be limited.

This disclosure also describes examples in which processing circuitry such as processing circuitry of a programmer, processing circuitry of server 110, or a combination thereof, may be configured to calculate an epoch-based therapy schedule based on a programmed therapy schedule of a programmer. In some examples, a timing system of an IMD, such as IMD 102, may have an epoch-based timing system which includes a counter that counts from a predetermined epoch date. Thus, processing circuitry may be configured to convert the real-time-based format of the programmed therapy schedule to an epoch-based format and deliver an epoch-based therapy schedule to the IMD. The IMD is then able to deliver therapy according to the programmed therapy schedule. Processing circuitry may use one or more algorithms to convert between a real-time based schedule and an Epoch-based timing system of an IMD and vice versa.

Moreover, in some examples, processing circuitry may be configured to receive, from the IMD, an epoch-based record of actual therapy delivered to a patient 106. Processing circuitry may be configured to calculate a real-time-based record of actual therapy delivered. The processing circuitry may also be configured to generate, for display, the record of actual therapy delivered. One or metrics, such as metrics of how a patient has adhered to a programmed therapy schedule (i.e., a "therapy adherence metric") may be calculated from the record of actual therapy delivered.

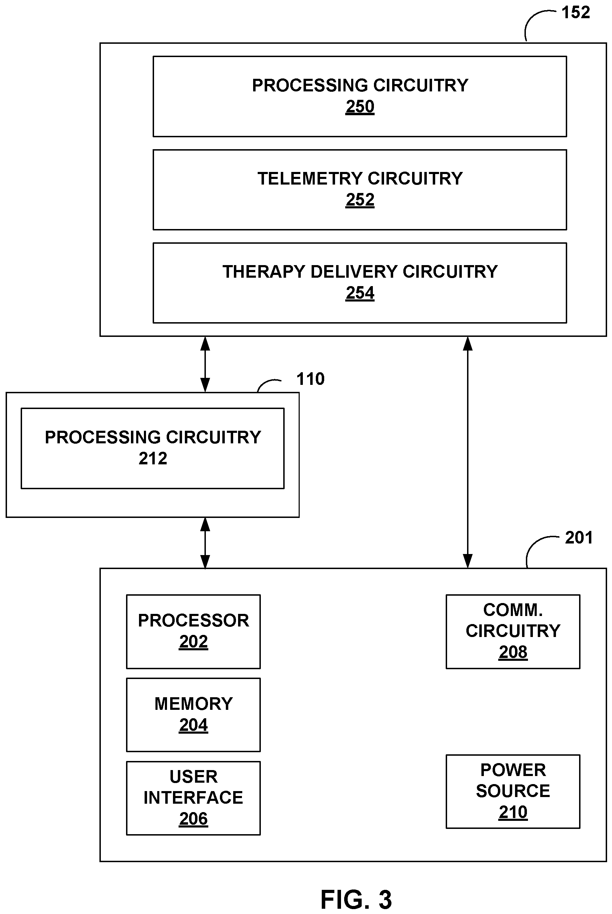

FIG. 3 is a block diagram of an example patient programmer, an implantable medical device, and a server according to one or more examples. Accordingly, and referring to FIG. 3, IMD 152 can include processing circuitry 250, telemetry circuitry 252, and therapy delivery circuitry 254. IMD 102 may include similar components as IMD 152. For ease the examples are described with IMD 152, but the example techniques are extendable to other examples of IMDs, including IMD 102.

In one or more examples, as will be understood by one of ordinary skill in the art, processing circuitry 250 comprises hardware and/or software to implement the functions of IMD 152. In one or more examples, telemetry circuitry 252 comprises hardware and/or software to communicate with, for example, programmer 201. In one or more examples, therapy delivery circuitry 254 is configured to provide electrical stimulation therapy proximate a nerve of a patient (e.g., sacral nerve(s) or tibial nerve).

The electrical stimulation therapy can be used to treat an impaired physiological function such as a bladder dysfunction or bowel dysfunction of patient 106. In one or more examples, the electrical stimulation therapy is implemented by an electrical current, or optionally, an electrical voltage. The electrical stimulation therapy may be defined by a plurality of parameters such as amplitude (e.g., current or voltage amplitude), pulse width, and/or frequency. Example ranges for the amplitude, pulse width, and/or frequency are provided below. In addition, electrical stimulation therapy may be defined by parameters such as duty cycle (e.g., on-time and off-times). For instance, a duty cycle may define a therapy session (e.g., on-time) during which patient 106 is receiving stimulation therapy defined by the amplitude, pulse width, and/or frequency. After the therapy session, there may be an amount of time (e.g., off-time), during which therapy is not being delivered. In some examples, the electrical stimulation therapy may also be defined by parameters that define when a therapy session is to start (e.g., start of on-time), for how long the therapy session is to last (e.g., on-time), for how long no therapy is delivered (e.g., off-time), on which days there is to be a therapy session, and on which days no therapy is delivered, etc.

Programmer 201 is communicatively coupled to IMD 152 via wireless communication. One example of programmer 201 is physician/clinician programmer 108. Another example of programmer 201 is patient programmer 104.

Referring to FIG. 3, a block diagram of programmer 201 is depicted, according to one or more examples. Programmer 201 can be a device for inputting information relating to patient 106, receiving information from IMD 152, and updating IMD 152. In some examples, such as where programmer 201 is patient programmer 104, programmer 201 can be a wearable communication device, with a therapy request input integrated into a key fob or a wristwatch, handheld computing device, smart phone, computer workstation, or networked computing device. Practically, programmer 201 can be a bring-your-own device provided by the patient, or provided by the healthcare provider in connection with the implantable.

In some examples, such as where programmer 201 is physician/clinician programmer 108, programmer 201 may be tablet computing device that is preloaded with a specific application to interface with IMD 152. The physician or clinician may interact with programmer 201 for programming IMD 152. As described in more detail, the physician or clinician may utilize examples of a workflow to program IMD 152, as well as view information about the usage of IMD 152.

Programmer 201 generally comprises a processor 202, a memory 204, a user interface 206, communication circuitry 208, and a power source 210. Processor 202 can be any programmable device that accepts digital data as input, is configured to process the input according to instructions or algorithms, and provides results as outputs. In an example, processor 202 can be a central processing unit (CPU) configured to carry out the instructions of a computer program. Processor 202 is therefore configured to perform at least basic arithmetical, logical, and input/output operations. In one or more examples, processor 202 correspond to individual hardware units, such as microprocessors, ASICs, DSPs, FPGAs, or other hardware units. In other examples, processor 202 can correspond to multiple individual hardware units, such as microprocessors, ASICs, DSPs, FPGAs, or other hardware units. Processor 202 may include processing circuitry configured to perform the processes discussed with respect to processor 202 (which may also be referred to herein as "processing circuitry 202").

Memory 204 can comprise volatile or non-volatile memory as required by the coupled processor 202 to not only provide space to execute the instructions or algorithms, but to provide the space to store the instructions themselves. In one or more examples, volatile memory can include random access memory (RAM), dynamic random-access memory (DRAM), or static random access memory (SRAM), for example. In one or more examples, non-volatile memory can include read-only memory, flash memory, ferroelectric RAM, hard disk, floppy disk, magnetic tape, or optical disc storage, for example. The foregoing lists in no way limit the type of memory that can be used.

User interface 206 can include a button or keypad, lights, a speaker for voice commands, a knob able to turn, a display, such as a liquid crystal (LCD), light-emitting diode (LED), or cathode ray tube (CRT). In some examples, the display may be a touch screen. Processor 202 can present and receive information relating to electrical stimulation and resulting therapeutic effects via user interface 206. For example, processor 202 can receive patient input via user interface 206. The input can be, for example, in the form of pressing a button on a keypad or selecting an icon from a touch screen. Processor 202 can also present information to the patient in the form of alerts related to delivery of the electrical stimulation to patient 106 or a caregiver via user interface 206.

Communications circuity 208 is configured to interface with IMD 152 and optionally, server 110 (FIG. 1), as will be described. Communications circuity 208 supports wireless communication between IMD 152 and, optionally, between server 110 and programmer 201 under the control of processor 202. Communications circuity 208 can also be configured to communicate with another computing device via wireless communication techniques, or direct communication through a wired connection. Communications circuity 208 can provide wireless communication via an RF or proximal inductive medium. In some examples, communications circuity 208 can include an antenna, which may take on a variety of forms, such as an internal or external antenna.

Examples of local wireless communication techniques that may be employed to facilitate communication between programmer 201 and another computing device include RF communication according to the 802.11 or Bluetooth specification sets, infrared communication, e.g., according to the IrDA standard, or other standard or proprietary telemetry protocols. In this manner, other external devices may be capable of communicating with programmer 201 without needing to establish a secure wireless connection.

Power source 210 delivers operating power to the components of programmer 201. Power source 210 can include a battery and a power generation circuit to produce the operating power. In some examples, the battery may be rechargeable by for example, an exterior power source.

Accordingly, as described, programmer 201 allows the user (e.g., patient, caretaker, clinician, physician) to program a therapy schedule and adjust therapy parameters (e.g., amplitude, frequency, and/or pulse width). Further, programmer 201 can communicate with IMD 152 to update the functionality of IMD 152.

Referring again to FIG. 1, system 100 may further comprise a physician programmer 108. Physician programmer 108 can be substantially similar to patient programmer 104, but be configured for physician operation or control. Accordingly, physician programmer 108 can likewise update the functionality of IMD 152, program therapy parameters, and program therapy schedule similar to the description above of programmer 201. In some examples, patient programmer 104 may be constrained in the amount and types of changes patient programmer 104 can make (e.g., maximum and minimum changes to value of therapy parameters). Physician programmer 108 may provide additional flexibility to the physician or clinician in setting parameters that may not be available with patient programmer 104.

As illustrated in FIG. 1, system 100 further optionally comprises a server 110. Server 110 can include one or more servers in a cloud computing environment. Server 110 can be configured to communicate with patient programmer 104, physician programmer 108 and/or IMD 102, 152 (not shown for ease of illustration) via wireless communication through a network access point. Server 110 can be co-located with patient programmer 104 or physician programmer 108, or located elsewhere, such as in a cloud computing data center.

Server 110 is shown as an example. In some examples, there may be a plurality of servers that form a cloud computing environment, and the example techniques described for server 110 may be performed by one or more of the servers of the cloud computing environment.

The cloud computing environment (e.g., just server 110 or a plurality of servers) include processing circuitry 212 (FIG. 3) and memory. The physician or clinician may access the cloud computing environment through programmer 108, and patient 106 may access the cloud computing environment through programmer 104. The processing circuitry of the cloud computing environment can be programmed by a physician user, including by physician programmer 108 or by another interface to the cloud computing environment. Server 110 or a combination of servers can store stimulation regimens, therapy parameters, and/or patient population statistics in response to such stimulation regimens and therapy parameters to guide future therapies of IMD 102. In some examples, a user can also interact with patient programmer 104, physician programmer 108, server 110 and/or IMD 102 remotely via a networked computing device. Although the above example in FIG. 1 is described with respect to IMD 102, the above examples may be applicable to IMD 152 to FIG. 2.

Moreover, in some examples, processing circuitry 212 and memory of server 110 or more generally one or more servers of the cloud computing environment may be configured to perform example techniques described in this disclosure, and possibly in combination with processing circuitry 250 or processor 202. For instance, processing circuitry 212 may receive information indicative of actual therapy delivered to patient 106, and may determine a therapy usage pattern and modifications to a programmed therapy schedule. As another example, processing circuitry 212 may be configured to receive information about a programmed therapy schedule from programmer 201 and convert the programmed therapy schedule into an epoch-based timing for programming IMD 152.

In some examples, the various operations described above may be shared by one or more of processing circuitry 212, processing circuitry 250, and processor 202. Therefore, in this disclosure, example techniques described by processing circuitry include examples where one or more of processing circuitry 212, processing circuitry 250, and processor 202, alone or in combination, perform the example techniques.

That is, in some examples, the various processing operations and components described herein can be implemented on IMD 102 or IMD 152. In some examples, the various processing operations and components described herein can be implemented on patient programmer 104. In some examples, the various processing operations and components described herein can be implemented on server 110. In some examples, the various processing operations and components described herein can be implemented on physician programmer 108. In some examples, the various processing operations and components described herein can be split between multiple components, such as patient programmer 104, physician programmer 108, 110, and/or IMD 102 or IMD 152.

In accordance with one or more examples described in this disclosure, IMD 102 and/or IMD 152 may be configured to deliver electrical stimulation therapy to the patient to address incontinence (e.g., urinary or fecal incontinence). Examples of the incontinence include urge urinary incontinence (UUI), urinary frequency (UF), fecal incontinence (FI), and other pelvic floor indications that can be treated with tibial neuromodulation.

In one or more examples, IMD 152 may be configured to deliver different amounts of electrical stimulation therapy based on a therapy period. For instance, during an implant period (e.g., as part of implanting IMD 152 or shortly after implanting IMD 152 while the patient is in clinic), IMD 152 may determine a set of stimulation parameters to control therapy delivery circuitry 254. As one example, the set of stimulation parameters may be according to a sensory threshold (e.g., a level at which the patient experiences paresthesia or otherwise "feels" the therapy) and/or a motor threshold (e.g., a level at which there is muscle movement due to the therapy or an electromyography (EMG) signal). For example, during or after implant, the HCP may increase the amplitude, pulse width, and/or frequency from a baseline level until the patient indicates that he or she feels the therapy. The HCP may use physician programmer 108 to output the therapy parameters that resulted in the patient feeling the therapy to IMD 152. To determine the set of stimulation parameters, IMD 152 may receive the set of stimulation parameters from physician programmer 108.

In some examples, the stimulation parameters may be based on sensed signals, such as sensed evoked compound action potential (eCAP) or electromyography (EMG) signals. For instance, therapy delivery circuitry 254 may output a signal that evokes a compound action potential (e.g., eCAP signal). Sensing circuitry of IMD 152 may sense the eCAP, and compare the eCAP to a threshold. Based on the comparison, IMD 152 may determine stimulation parameters.

After implant, it may be possible for IMD 152 to start delivering therapy in accordance with the set of stimulation parameters (e.g., those determined according to a sensory and/or motor threshold). However, it may not be necessary to keep therapy at the initial set of stimulation parameters. Rather, over time, it may be possible to reduce the amount of electrical stimulation therapy that is delivered, as the patient may experience therapeutic benefits even with lower electrical stimulation therapy. The amount of electrical stimulation therapy may refer to the energy of pulses (e.g., amplitude, frequency, pulse width), therapy session occurrence, and therapy session duration.

For instance, after implant, during an induction period, IMD 152 may be configured to deliver electrical stimulation therapy at a relatively higher stimulation regimen. For instance, IMD 152 may be configured to deliver electrical stimulation therapy for a relatively extended period of time (e.g., for an extended therapy session duration), may be configured to deliver electrical stimulation therapy relatively often (e.g., for a relatively higher rate of therapy session occurrence), and/or may be configured to deliver electrical stimulation therapy at a relatively higher energy level, e.g., with a relatively higher amplitude. In some examples, the induction period may be started on the first day of the implant procedure, eliminating the need for a follow up visit to program IMD 152.

Based on patient outcomes or patient goals, after the induction period, IMD 152 may be configured to operate in accordance with a maintenance period. In the maintenance period, the amount of electrical stimulation delivered to the patient may be reduced, without impact on effectiveness of therapy. For example, there may be a reduction in a therapy session duration (e.g., a therapy session duration in the maintenance period is less than a therapy session duration of the induction period stimulation parameters). As an example, if the therapy session duration during the induction period is four hours of stimulation, then in the maintenance period the therapy session duration may be 30 minutes.

As another example, there may be a reduction in a therapy session occurrence (e.g., a therapy session occurrence in the maintenance period is less than a therapy session occurrence of the induction period stimulation parameters). As an example, if the therapy session occurrence during the induction period is every day, then in the maintenance period the therapy session occurrence may be once a week or once every two weeks.

In some examples, the stimulation parameters in the maintenance period may include a reduction in both the therapy session duration and the therapy session occurrence. For example, if the therapy session duration was four hours in the induction period, and the therapy session occurrence was once a day, then in the maintenance period, the therapy session duration may be 30 minutes (or less), and the therapy session occurrence may be once every week or once every two weeks.

The above examples of 30 minutes of stimulation every week or every other week is provided as an example only. In some examples, the range of therapy session duration may be 1 minute to 1 hour (e.g., range of 1 minute to 10 minutes, 10 minutes to 15 minutes, 15 minutes to 30 minutes, or 30 minutes to one hour), and the range of the therapy session occurrence may be once a day to once a month (e.g., once a day, once every two days, once every three days, once every four days, once every five days, once every six days, once a week, once every two weeks, once a month, and any time in between). The therapy session duration and/or therapy session occurrence may be different for induction and maintenance periods. However, other ranges are possible, and the techniques are not limited to the above example ranges. Moreover, there may be additional other ways to reduce stimulation such as by reducing amplitude of the stimulation.

Also, in the above examples, there is one maintenance period. However, the example techniques are not so limited. For instance, in some examples, there may be a plurality of maintenance periods, and during respective checkpoints, the patient may indicate whether he or she is experiencing the desired patient outcomes. If the patient is experiencing the desired patient outcomes, during a first maintenance period, IMD 152 may be configured to deliver stimulation in accordance with a second maintenance period. For instance, there may be a set of stimulation parameters that therapy delivery circuitry 254 outputs, where the amount of electrical stimulation therapy that is delivered is less than the amount of electrical stimulation therapy that is delivered during the first maintenance period (e.g., in terms of therapy session duration, therapy session occurrence, stimulation amplitude, etc.).

In any induction period or the plurality of maintenance periods, processing circuitry (such as processor 202 of programmer 201 or processing circuitry 212) may determine a therapy usage pattern over a plurality of instances of therapy delivery. The processing circuitry (such as processor 202 of programmer 201 or processing circuitry 212) may receive a first set of information, which includes information, or a record, of therapy actually delivered to the patient. This first set of information may include both the time, date, and duration of delivered therapy, but also patient information regarding whether he or she is experiencing the desired patient outcomes.

Processing circuitry (such as processor 202 of programmer 201 or processing circuitry 212) may be able to automatically generate a modification to a programmed (i.e., prescribed) therapy schedule, whether in any induction or maintenance schedule, based on the therapy usage pattern. The recommended modification may be determined solely based on the therapy usage pattern, but may also include comparing information of actual therapy delivered to a patient (i.e., a first set of information) and a programmed therapy schedule for a patient (i.e., a second set of information).

The following examples illustrate various user interfaces and techniques for managing electrical stimulation as described above. Programmer 201 (e.g., patient programmer 104, physician programmer 108, or another external computing device) may output the user interfaces and screens described herein.

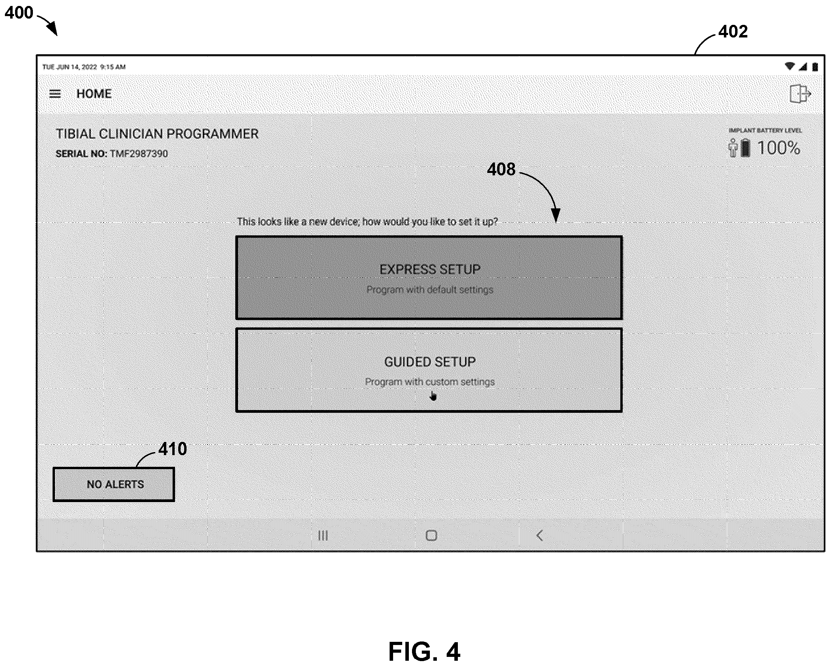

FIG. 4 is a conceptual diagram illustrating an example home screen 402 for navigating within an example user interface 400 when the IMD has not been previously activated or used for therapy. User interface 400 may include several different screens as the user can navigate to different functions to view sensed information, view stored data, or adjust various stimulation parameter values. Alert button 410 shows "no alerts" because there are no alerts to be shown. However, if there are alerts for the user, alert button 410 may indicate that there are alerts, or the number of alerts, and alert button 410 may be selectable to cause user interface 400 to show a list of the alerts for the user.

The home screen 402 in FIG. 4 may also include a menu 408 that includes several selectable buttons that enable a user to choose between various setup modes. The selectable buttons of menu 408 may include "Express Setup" and "Guided Setup." Physician programmer 108 may switch to the appropriate screen in response to user selection of the respective selectable button. Either of the "Express Setup" or "Guided Setup" buttons of menu 408 takes the user to screens associated with selecting electrode configurations for stimulation. The "Express Setup" option of menu 408 generally allows for quicker setup with less customization of relevant stimulation parameters, and may load default stimulation parameters. The "Guided Setup" option of menu 408, as will be discussed in more detail, generally allows for more customization of relevant stimulation parameters. The "Guided Setup" option of menu 408 may include more setup steps compared to the "Express Setup" option.

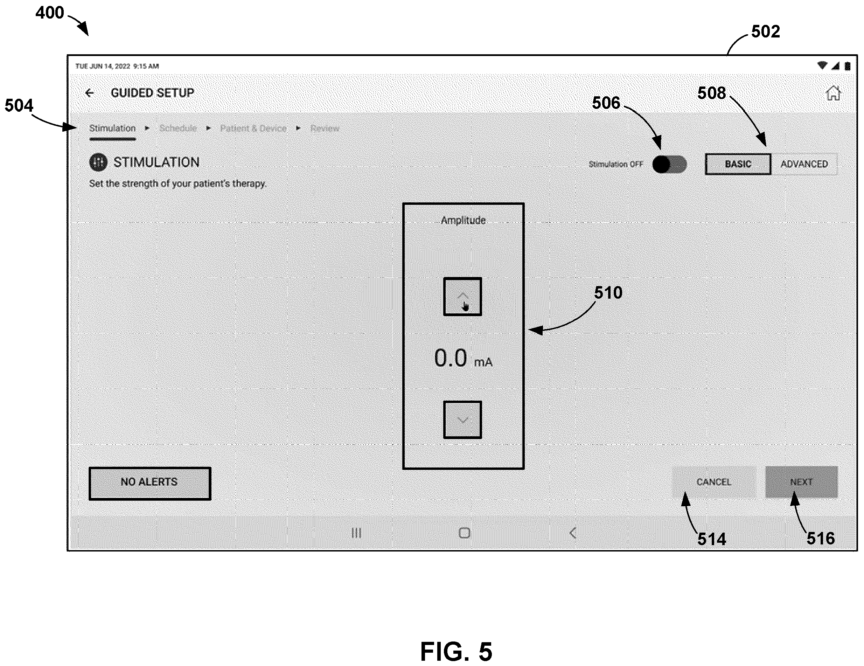

FIGS 5-10 are conceptual diagrams illustrating example setup screens for managing stimulation therapy parameters. FIG. 5 is a conceptual diagram illustrating an example setup screen for managing stimulation amplitude. In the example of FIG. 5, screen 502 has been entered to begin the "Guided Setup" process. Screen 502 allows for selection of stimulation amplitude. Menu 504 indicates the current stage in the setup, which is shown as "Stimulation" in FIG. 5 As the user moves through the different stages of the setup, the respective stage is underlined to show the current status. Stimulation toggle switch 506 may be provided in some, most, or all of the different screens within user interface 400 to enable the user to turn stimulation on or off at any time. Stimulation toggle switch 506 may also indicate to a user whether the IMD is currently providing stimulation therapy. Menu 508 includes a "Basic" button and a "Advanced" button. The "Basic" option generally allows for a streamlined setup, which in some examples may include only where a user sets stimulation amplitude. The "Advanced" option, discussed in relation to later figures, may allow for a more customizable setup in which a user sets more stimulation parameters including amplitude, pulse width, pulse rate, patient limits which limits the ability of patients to adjust therapy settings, and a "Soft Start / Stop" option.

In the example of FIG. 5, which includes a default selection of the "Basic" option of menu 508, amplitude selection menu 510 may include two selectable buttons allowing a user to increase or decrease stimulation amplitude. The selectable buttons of amplitude selection menu 510 may include an "up" arrow button and a "down" arrow button. When a user selects the "up" arrow button and the "down" arrow button, the stimulation amplitude may increase or decrease by any suitable increment, such as 0.1 mA, 0.2 mA, 0.5 mA, 1 mA, or another suitable increment. Menu 510 may also include a display of the current set stimulation amplitude (shown as "0.0 mA" in the example of FIG. 5).

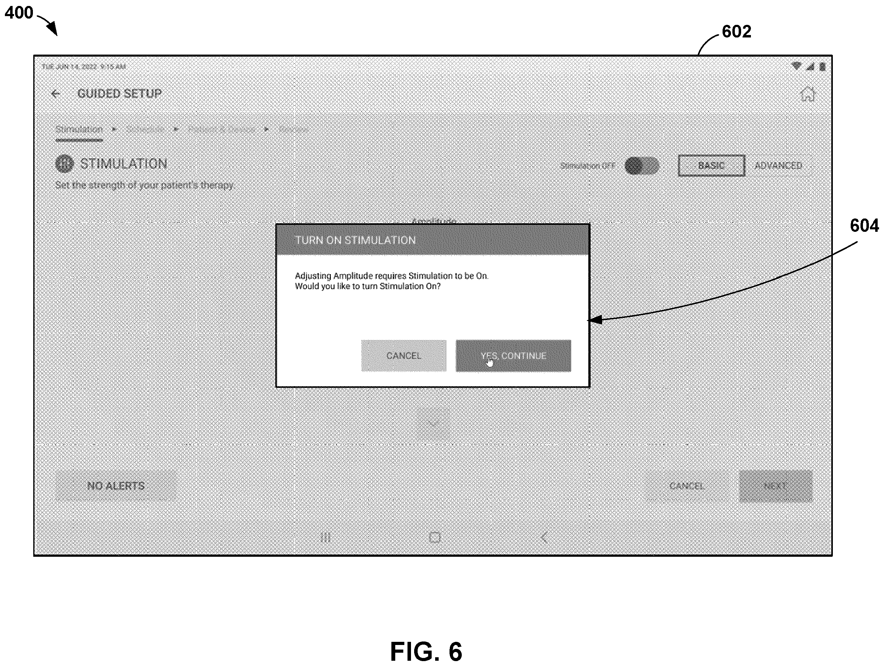

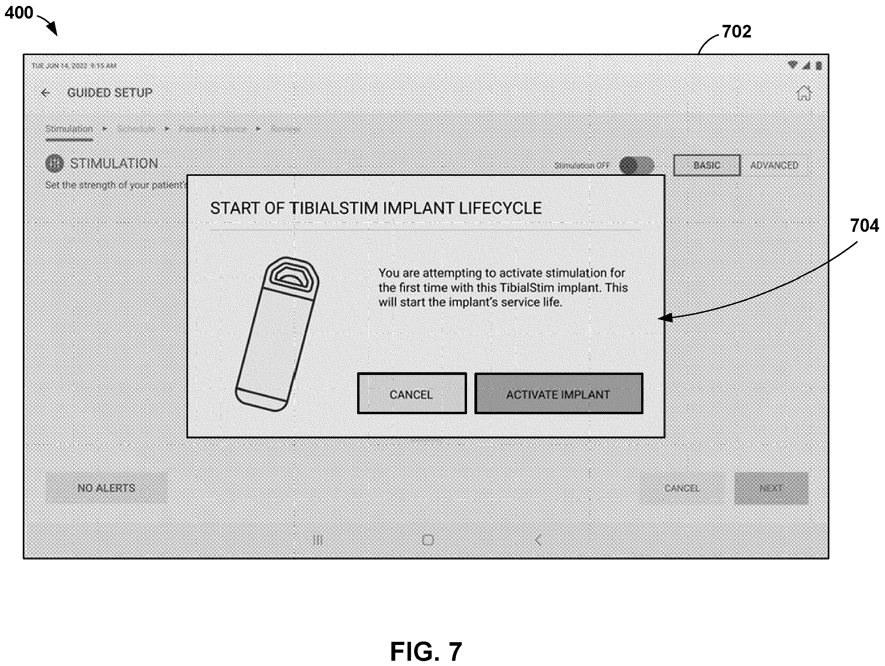

FIG. 6 is a conceptual diagram illustrating an example setup screen for IMD startup. When a user first presses the "up" arrow button of amplitude selection menu 510 of FIG. 5, a pop-up window shows screen 602 of FIG. 6 which includes example pop-up window 604 requesting the user to confirm the decision to initiate stimulation therapy. The IMD (e.g., IMD 152) may begin to deliver stimulation therapy in response to the user selecting the "Yes, Continue" button of pop-up window 604. FIG. 7 is a conceptual diagram illustrating an example setup screen for IMD startup. If the IMD has not been used before, or has otherwise been reset, a pop-up window shows screen 702 of FIG. 7 which includes example pop-up window 704 requesting the user to confirm the decision to activate the IMD (e.g., IMD 152) by selecting the "Activate Implant" button. The IMD (e.g., IMD 152) will begin to deliver stimulation therapy in response to the user selecting the "Activate Implant" button of pop-up window 704.

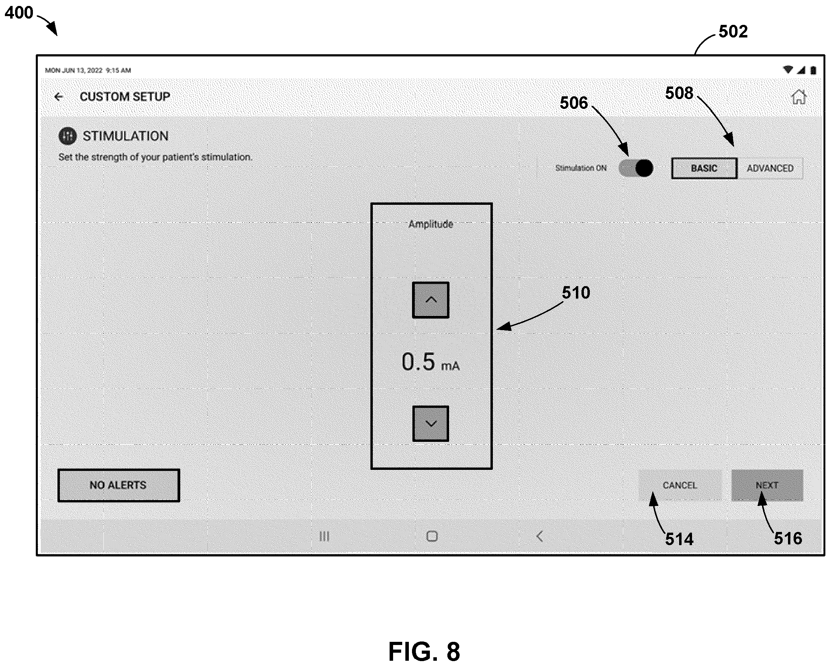

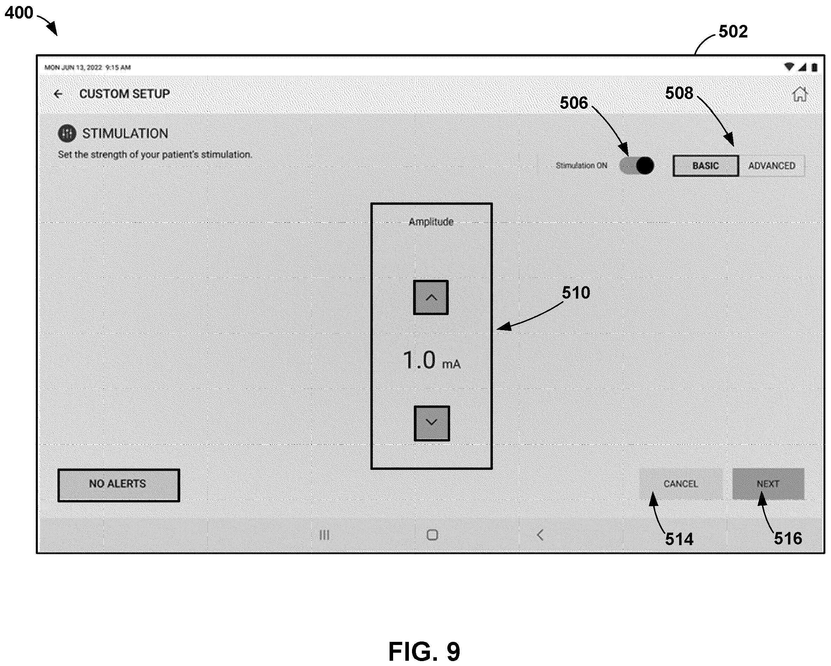

FIG. 8 is a conceptual diagram illustrating an example setup screen for managing stimulation amplitude. In the example of FIG. 8, screen 502 shows a plurality of selectable therapy parameter control icons. The processing circuitry (e.g., processor 202) of programmer 201 may be configured to output the plurality of selectable therapy parameter control icons on a display, such as on user interface 400. The icons may include stimulation toggle switch 506. Switch 506 is shown in in the "Stimulation On" position, indicating that therapy is being delivery by IMD 152. Additionally, in the example of FIG. 8, the icons may include amplitude selection menu 510 displays a stimulation amplitude of "0.5 mA." Screen 502 of FIG. 8 may be the result of the user's initiation of stimulation therapy as described with respect to FIGS. 5-7. FIG. 9 is a conceptual diagram illustrating an example setup screen for managing stimulation amplitude. FIG. 9 shows amplitude selection menu 510 displaying a stimulation amplitude of "1.0 mA," which may be the result of the user pushing the "up" arrow button of amplitude selection menu 510 in FIG. 8.

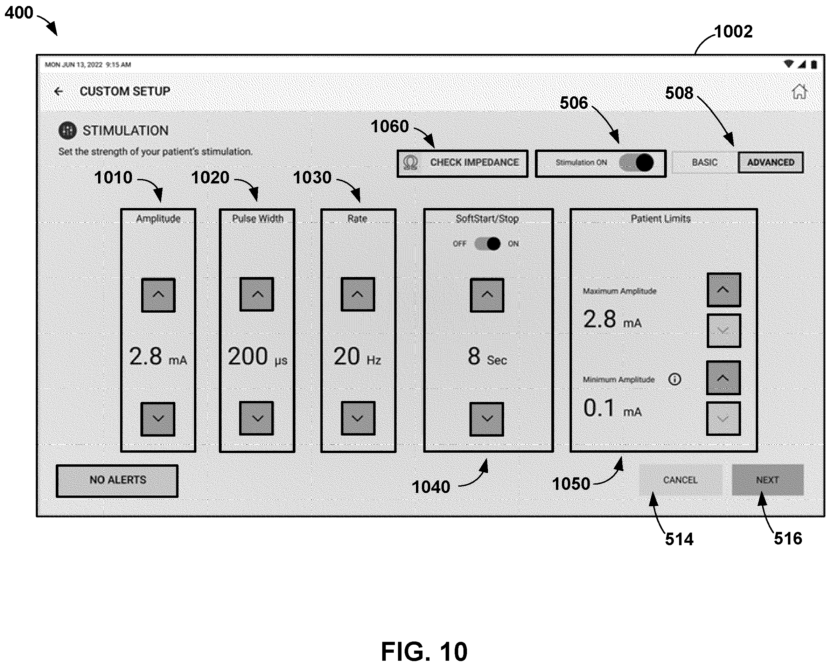

FIG. 10 is a conceptual diagram illustrating an example advanced setup screen for configuring stimulation. The example of FIG. 10 shows screen 1002, which is the result of a user selecting the "Advanced" button of menu 508 (such as in the example of FIG. 5). Screen 1002 may include the therapy stimulation parameter choice buttons similar to screen 502, as well as additional parameters and buttons. For example, screen 1002 may include amplitude selection menu 1010 which may include an "up" arrow button and a "down" arrow button. Amplitude selection menu 1010 may also include a display of the current set stimulation amplitude (shown as "2.8 mA" in the example of FIG. 10). Amplitude selection menu 1010 may the same or substantially similar to amplitude selection menu 510. Screen 1002 may include pulse width selection menu 1020 which may include an "up" arrow button and a "down" arrow button. Pulse width selection menu 1020 may also include a display of the current set pulse width (shown as "200 µs" in the example of FIG. 10). Screen 1002 may include rate selection menu 1030 which may include an "up" arrow button and a "down" arrow button. Rate selection menu 1030 may also include a display of the current set rate (shown as "20 Hz" in the example of FIG. 10). Screen 1002 may include "SoftStart/Stop" menu 1040 which may include an "up" arrow button and a "down" arrow button. "SoftStart/Stop menu" 1040 may also include a display of the current set duration for the "SoftStart/Stop" duration (shown as "8 Sec" in the example of FIG. 10). Screen 1002 may "Patient Limit" menu 1050 which may include an "up" arrow button and a "down" arrow button for a "Maximum Amplitude" parameter as well as an "up" arrow button and a "down" arrow button for a "Minimum Amplitude" parameter. "Patient Limit" menu 1050 may also include a display of the current set patient "Maximum Amplitude" parameter (shown as "2.8 mA" in the example of FIG. 10) as well as a display of the current set patient "Minimum Amplitude" parameter (shown as "0.1 mA" in the example of FIG. 10). "Patient Limit" menu 1050 may include additional buttons or options for a user to restrict the parameters that a patient may change. For example, "Patient Limit" menu 1050 may include options for restricting a patient's ability to skip single therapy sessions, or change the schedule of prescribed/programmed therapy schedule. Some examples may include restricting or allowing a patient to change the time of day that therapy starts, or change the day of the week that therapy occurs. Screen 1002 may also include a "Check Impedance" button 1060, which allows a user to test the impedance of the IMD. Screen 1002 may include other buttons similar to those from FIGS. 5-9.

Once the stimulation parameters have been selected (such as on screen 502 of FIGS. 5-9 or screen 1002 of FIG. 10), the user can select "NEXT" button 516 to move to the next screen to establish a therapy schedule. Alternatively, the user can select "Cancel" button 514 to return to a "Home" screen (such as screen 402 of FIG. 4) or another previous screen.

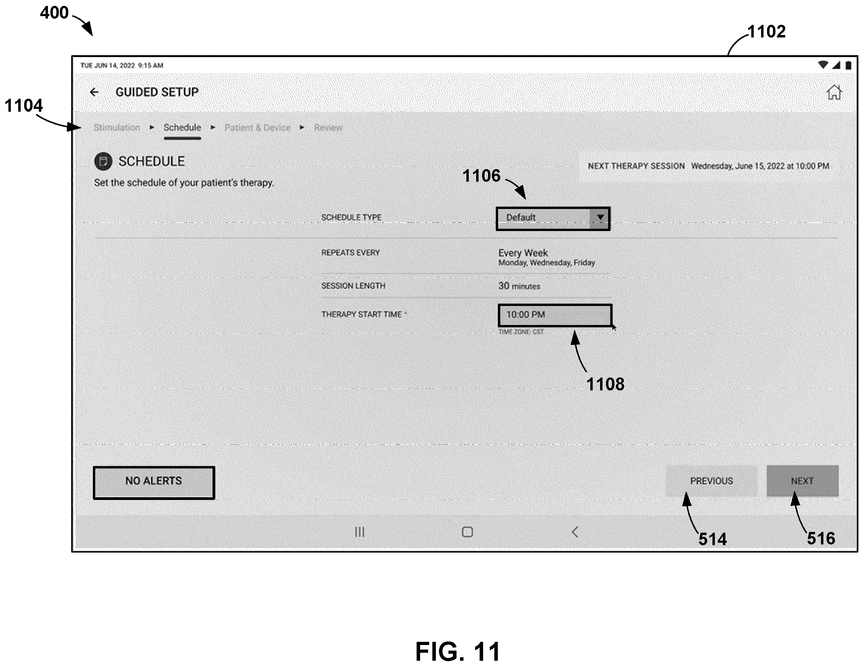

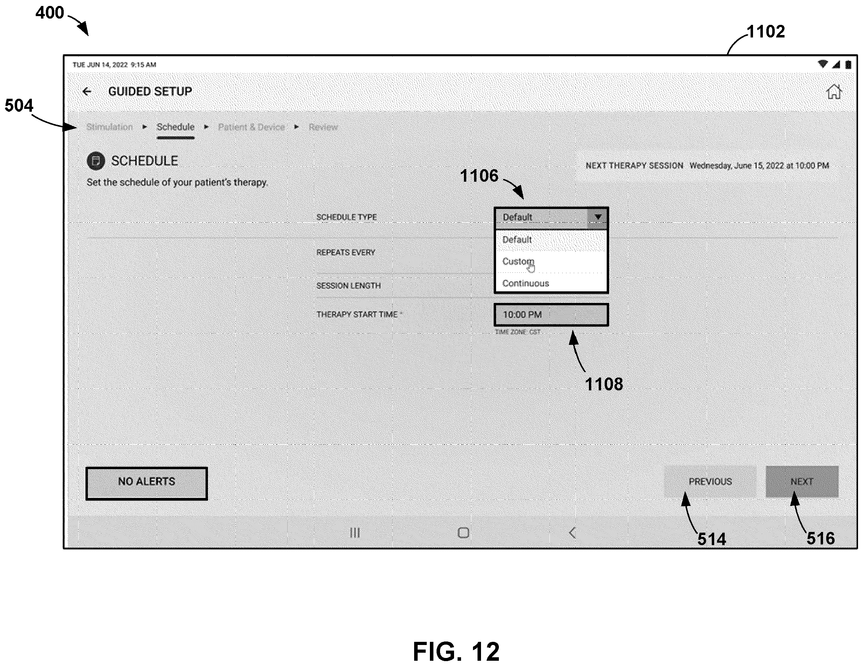

FIGS. 11-19 are conceptual diagrams illustrating example setup screens for managing stimulation therapy schedules. FIG. 11 is a conceptual diagram illustrating an example setup screen for managing a stimulation schedule. In the example of FIG. 11, screen 1102 allows for selection of stimulation schedule parameters. Menu 1104 indicates the current stage in the setup, which is shown as "Schedule" in FIG. 11. Screen 1102 includes drop-down menu 1106 from which a user can select a "Schedule Type." Screen 1102 illustrates an example where drop-down menu 1106 indicates a "Default" choice for "Schedule Type." When drop-down menu 1106 indicates a "Default" choice for "Schedule Type," various parameters including the schedule repetition and session length may be provided on screen 1102. Therapy start time menu 1108 may provide a suggested therapy start time (shown as "10:00 PM" in FIG. 11), but a user may change the therapy start time in a time selection box of therapy start time menu 1108. FIG. 12 is a conceptual diagram illustrating an example setup screen for managing a stimulation schedule. In the example of FIG. 12, which show screen 1102, drop-down menu 1106 has been toggled to display various selectable schedule options such as "Default," "Custom," or "Continuous."

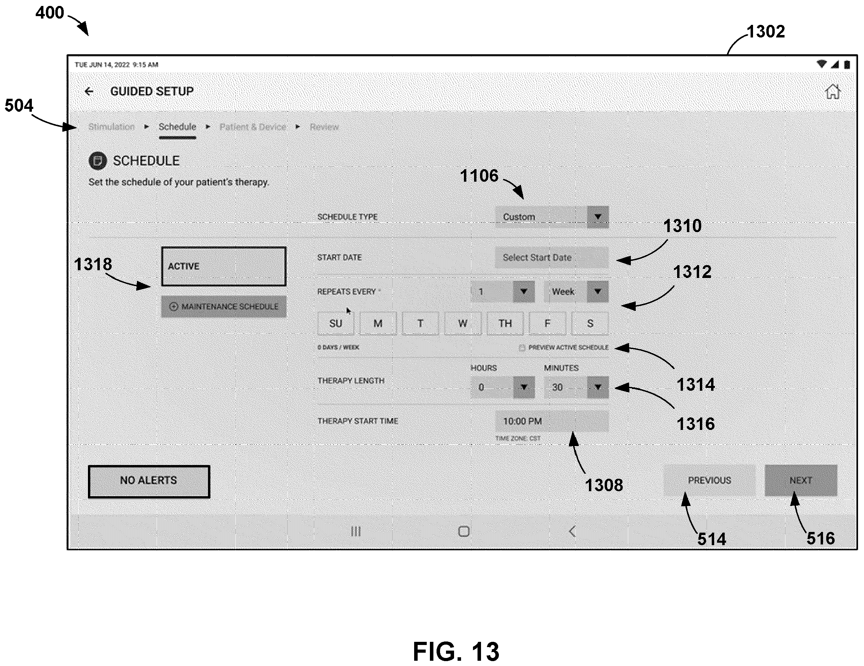

FIG. 13 is a conceptual diagram illustrating an example setup screen for managing a stimulation schedule. FIG. 13 shows example screen 1302, which is the result of a user selecting the "Custom" drop-down option of drop-down menu 1106 as shown in previous figures. Screen 1302 may include a plurality of selectable therapy schedule customization icons. The processing circuitry (e.g., processor 202) of the programmer 201 may be configured to output the plurality of selectable therapy schedule customization icons on a display, such as on user interface 400. Screen 1302 allows for selection of stimulation schedule parameters. For example, screen 1302 may provide therapy start time menu 1308, therapy start date menu 1310, repetition menu 1312, as well as therapy length menu 1316. Therapy start time menu 1308 may provide a suggested therapy start time (shown as "10:00 PM" in FIG. 13), but a user may change the therapy start time in a time selection box of therapy start time menu 1308. Similarly, therapy start date menu 1310 may allow a user to set a therapy start date in a date selection box of therapy start date menu 1310. The therapy start date menu may allow for a start date of the current day, any day in the future, or any day in the past. Repetition menu 1312 includes multiple drop-down menus which allows a user to select a number of units (such as "1," "2," "3," etc.), as well as a durational increment ("days," "weeks," "months," etc.) to set a repetition schedule for therapy. Repetition menu 1312 also includes buttons for each day of the week (Monday, Tuesday, Wednesday, etc.) to allow a user to set specific days of the week for a repetitive schedule. Therapy length menu 1316 includes multiple drop-down menus which allows a user to select an increment of hours (such as "1," "2," "3," etc.) as well as an increment of minutes (such as "1," "2," "3," "15," "30," "45," etc.) to set a duration of therapy delivered during each therapy session. The foregoing menu options are illustrative examples and may be configured in other ways.

Screen 1302 also illustrates an example schedule type menu 1318 which may include buttons to allow for selection of various therapy schedule types. For example, menu 1318 may include a first schedule type such as "Active" or "Induction" therapy schedule, which may be presented as a default option or as a selectable button. Additionally, menu 1318 may have a button for the selection of additional therapy schedules, such as a "Maintenance Schedule." While the example of menu 1318 on screen 1302 contains a single button for each of the "active"/induction and maintenance schedules, menu 1318 may allow for selection and configuration of multiple induction, maintenance, or other schedules. For example, menu 1318 could allow buttons for day- or time-specific therapy schedules.

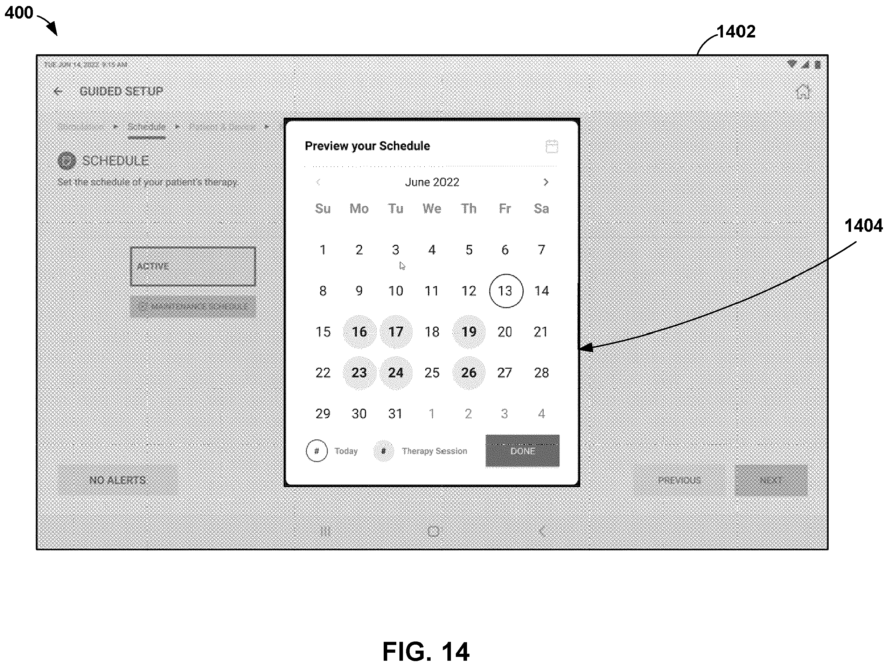

Screen 1302 also illustrates "Preview Active Schedule" button 1314 which allow a user to view an anticipated therapy schedule. FIG. 14 is a conceptual diagram illustrating an example setup screen for managing a custom stimulation schedule. FIG. 14 includes example pop-up window 1404 which displays a calendar view to preview a given therapy schedule. Example popup window 1404 includes a calendar view of a given single month to preview a given therapy schedule, although other ways of previewing a therapy schedule may include a weekly, multi-month, or yearly view. Example pop-up window 1404 can highlight the given days of a week and/or month on which therapy is scheduled.

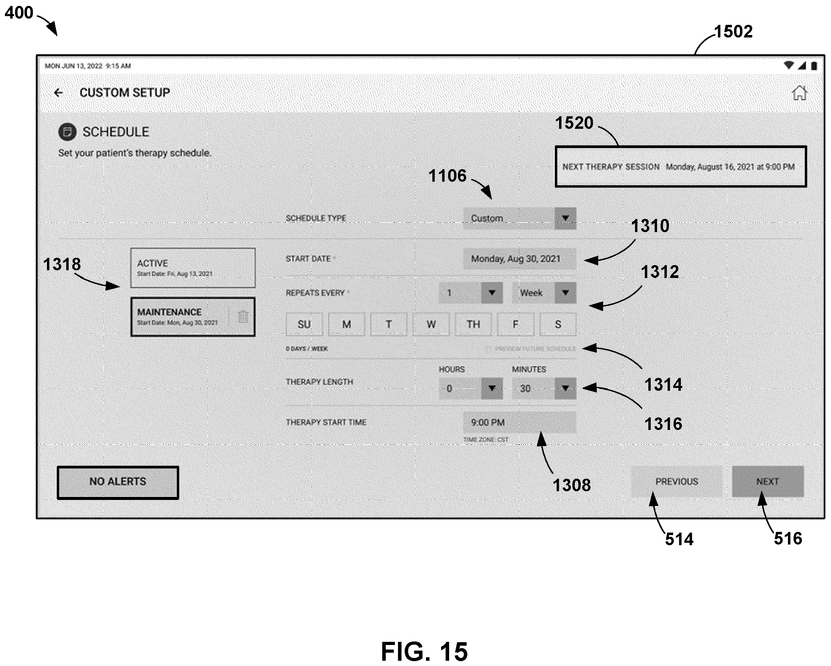

FIG. 15 is a conceptual diagram illustrating an example setup screen for managing a custom stimulation schedule. FIG. 15 shows example screen 1502, which is the result of a user selecting the "Maintenance" button of menu 1318 as discussed in connection with previous figures to create an additional therapy schedule. Screen 1502 may be the same or substantially similar to screen 1302. For example, screen 1502 may provide therapy start time menu 1308, therapy start date menu 1310, repetition menu 1312, as well as therapy length menu 1316, which are discussed in connection with screen 1302. When selecting a therapy start from therapy start date menu 1310 on screen 1502, the selected date may define a transition date between an "Active"/Induction schedule to a "Maintenance" schedule. The IMD may automatically switch between different therapy schedules on the date that defines the transition between the "Active"/Induction and "Maintenance" schedules. Screen 1502 may include example box 1520, which may display an indication of the next therapy schedule session of a patient. For instance, when an "active"/induction schedule has been established on screen 1302, box 1520 on screen 1502 may indicate to a user the date and time of the next scheduled therapy session, which may be in the future. While the example of FIG. 15 shows the creation of a single additional schedule after the "Active"/Induction schedule, the system may allow for creation of additional schedules beyond a single "Maintenance" schedule with unique stimulation parameters. Further, stimulation parameters may be customized for select times within a given therapy schedule, such as having specific stimulation parameter settings and therapy durations on given days of a week or at certain times of a day. Scheduled therapy can also be used in conjunction with traditional cycling therapy methods.

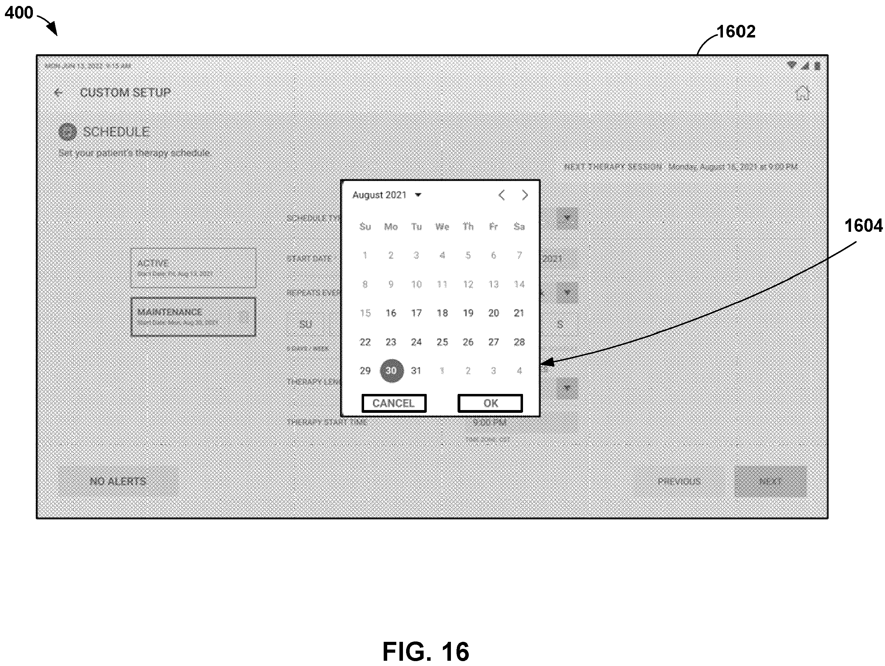

FIG. 16 is a conceptual diagram illustrating an example setup screen for managing a custom stimulation schedule. FIG. 16 shows screen 1602, which includes example pop-up window 1604 which displays a calendar view to allow a user to select a therapy start date after pressing the therapy start date menu 1310 of screen 1502. After selecting a therapy start date on pop-up window 1604, a user may select an "OK" button to confirm selection of the start date and close pop-up window 1604.

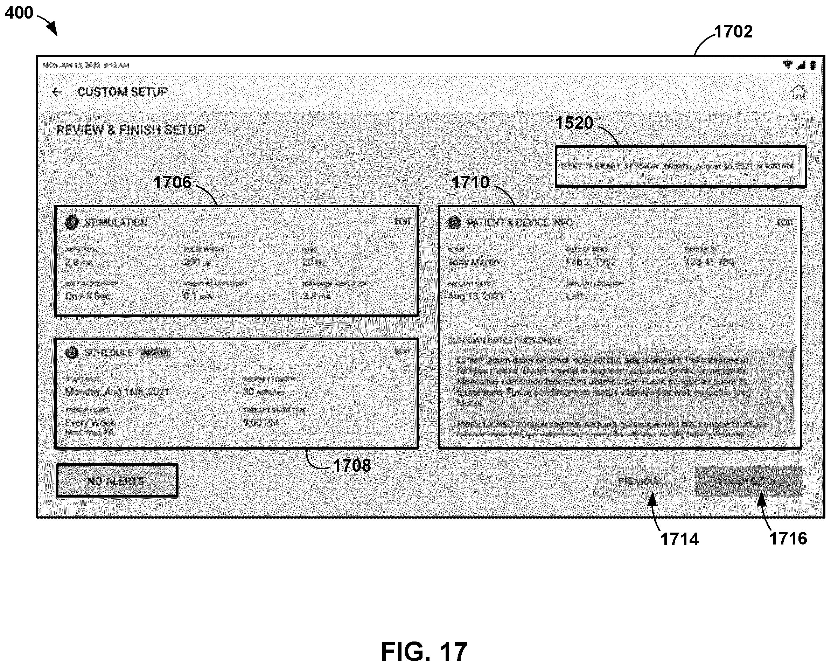

FIG. 17 is a conceptual diagram illustrating an example review screen for managing a stimulation schedule. FIG. 17 shows example screen 1702, which is the result of a user selecting the "Next" 516 button of screens 1302 or 1502. Screen 1702 may provide a summary of patient and programmed stimulation therapy information, which may be presented in different sections of screen 1702. For example, screen 1702 may provide "Stimulation" summary box 1706, "Schedule" summary box 1708, and "Patient & Device Info" summary box 1710. "Stimulation" summary box 1706 may include a summary of parameters chosen on previous screens such as stimulation amplitude, pulse width, rate, Soft start/stop duration, minimum amplitude, and maximum amplitude. "Schedule" summary box 1708 may include a summary of scheduling parameters chosen on previous screens such as start date, therapy length, therapy days (of the week), and therapy start time. "Schedule" summary box 1708 may include a summary of "default" therapy scheduling parameters. "Patient & Device Info" summary box 1710 may include a summary of relevant patient information input by a clinician or other user, as well as IMD information relevant to the specific patient, including the name, birth date, and a relevant patient identification number of the implanted patient. Other information in "Patient & Device Info" summary box 1710 may include date of implant and location (ex. "left" or "right"), as well as clinician notes. Each of the relevant information summary boxes, including "Stimulation" summary box 1706, "Schedule" summary box 1708, and "Patient & Device Info" summary box 1710, may include an "Edit" button which allows, when pressed, the user to return to the relevant previous configuration screen. Alternatively, pressing the "Edit" button in boxes 1706, 1708, of 1710 may present a pop-up window (not shown) allowing a user to edit any relevant parameters.

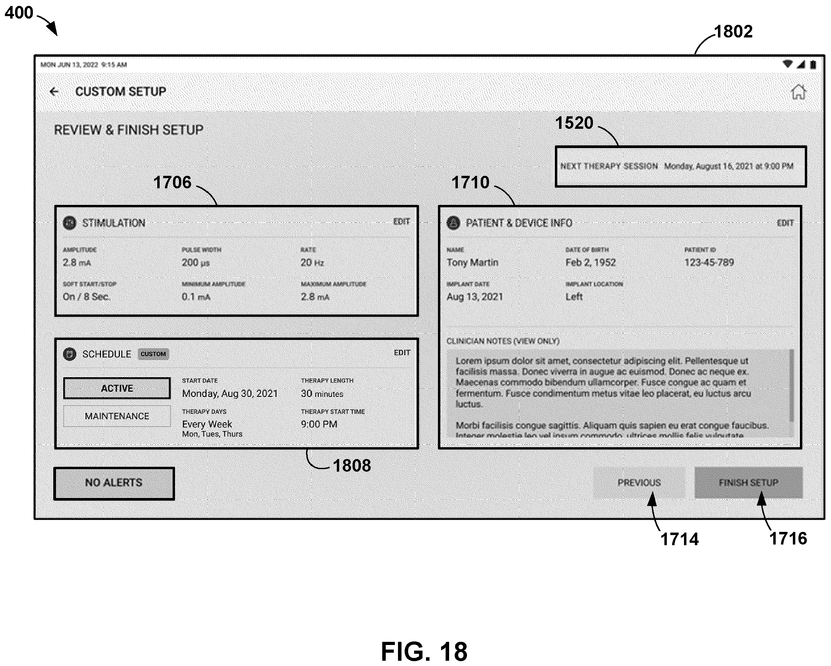

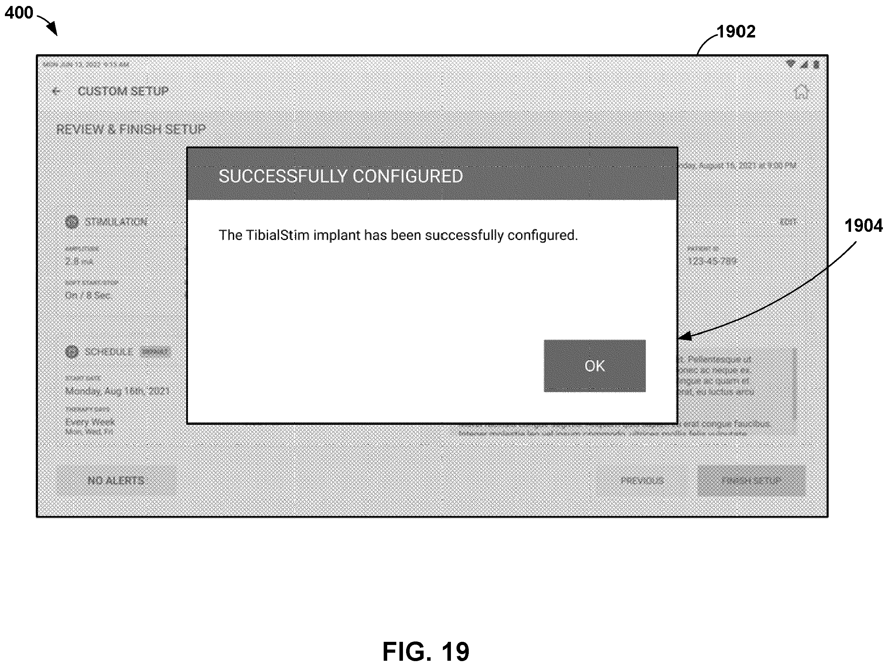

FIG. 18 is a conceptual diagram illustrating an example review screen reviewing and finishing device setup. FIG. 18 shows example screen 1802, which is similar to screen 1702, except includes "Schedule" summary box 1808 which may include a summary of "custom" therapy scheduling parameters (including options to view both "Active" / Induction and "Maintenance" schedules). Once a user has finished reviewing relevant information on screen 1702 and/or screen 1802, the user can select "FINISH SETUP" button 1716 to move to the next screen to finish setup of relevant therapy parameters. Alternatively, the user can select "PREVIOUS" button 1714 to return to previous screens. FIG. 19 is a conceptual diagram illustrating an example review screen for confirming device setup is complete. When the user selects "FINISH SETUP" button 1716, a pop-up window shows screen 1902 of FIG. 19 which includes example pop-up window 1904 notifying the user that the IMD has been successfully configured.

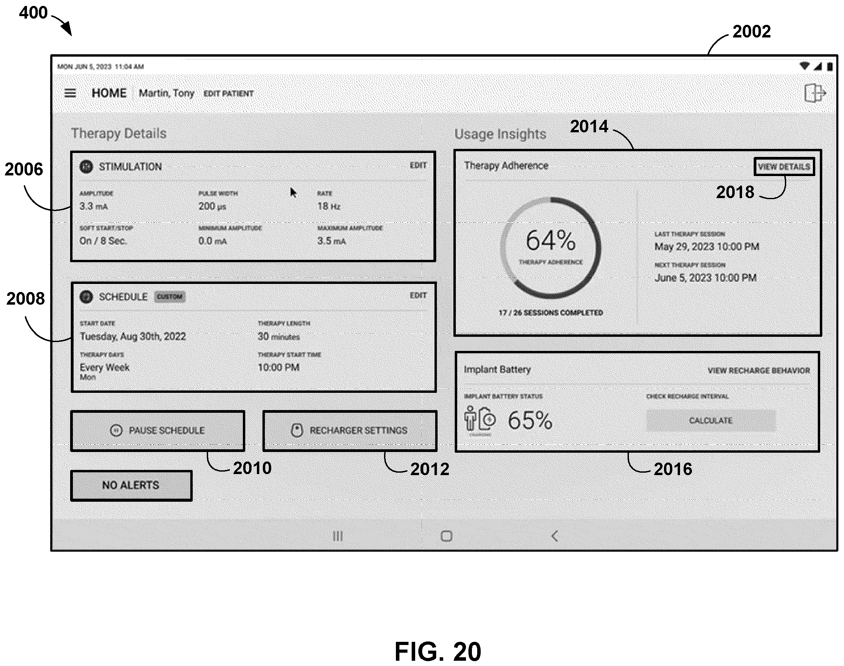

FIGS. 20-22 are conceptual diagrams illustrating example screens for reviewing diagnostic information provided by the system. FIG. 20 is a conceptual diagram illustrating an example home screen for providing a dashboard view. FIG. 20 illustrates an example home screen 2002 for navigating within an example user interface 400 when the IMD (e.g., IMD 152) has been implanted for a period of time after initial setup. Screen 2002 may provide a summary of patient and programmed stimulation therapy information, which may be presented in different sections of screen 2002. For example, screen 2002 may provide "Stimulation" summary box 2006, "Schedule" summary box 2008, and "Implant Battery" summary box 2016. "Stimulation" summary box 2006 may include a summary of parameters chosen on previous screens such as stimulation amplitude, pulse width, rate, Soft start/stop duration, minimum amplitude, and maximum amplitude. "Stimulation" summary box 2006 may be the same or substantially similar to "Stimulation" summary box 1706 as discussed in connection with above description. "Schedule" summary box 2008 may include a summary of scheduling parameters chosen on previous screens such as start date, therapy length, therapy days (of the week), and therapy start time. "Schedule" summary box 2008 which may include a summary of "custom" therapy scheduling parameters (including options to view both "Active" / Induction and "Maintenance" schedules). "Implant Battery" summary box 2016 may include information about the IMD (e.g., IMD 152) battery, such as the percentage of charge remaining in the IMD battery, and an option to check the recharge interval of the IMD battery.

In the example of FIG. 20, screen 2002 may also present actionable buttons for adjusting therapy. For example, "Pause Schedule" button 2010 allows a user to pause therapy delivery. This "pause Schedule" button 2010 may allow a user to pause all upcoming therapy sessions for a given therapy schedule, or to select a period of time in the future to pause or skip therapy sessions of a given therapy schedule. "Recharger Settings" button 2012 may allow a user to adjust IMD recharger settings, such as external recharger 154 discussed previously.

In the example of FIG. 20, screen 2002 may also present "Therapy Adherence" box 2014. Box 2014 may present a metric indicating a user's adherence to a scheduled therapy regimen. For example, box 2014 may display a chart, graph, or other visualization indicating a therapy adherence metric. In the example of FIG. 20, box 2014 displays a pie chart where the therapy adherence metric is a percentage indicating the number of sessions a user has fully completed as compared to the total amount of prescribed sessions. In general, therapy adherence metrics displayed in box 2014 may be calculated by comparing a user's actually delivered therapy to a user's prescribed therapy as programmed. The number of sessions actually delivered or completed for purposes of calculating a therapy adherence metric may be those therapy session that are fully completed (e.g. 100% of the prescribed/ programmed durational requirement) or those sessions that meet some durational threshold requirement (e.g. 90% of the prescribed/ programmed durational requirement). A therapy session may be considered not delivered or not complete where the patient pauses, skips, cancels, or otherwise alters the scheduled therapy session. The total number of prescribed sessions for the calculation may be the total amount of prescribed therapy sessions since the last programming or the device initialization. As an illustrative example, box 2014 displays a therapy adherence metric as 64%, which may be calculated based on an example number of completed sessions (17 sessions) divided by an example number of total prescribed sessions (26 session). Box 2014 may also display the date and time of the patient's last therapy session as well as the date and time of the next scheduled therapy session. Box 2014 may also include "View Details" button 2018, which may allow a user to view more specific details regarding a patient's therapy adherence.

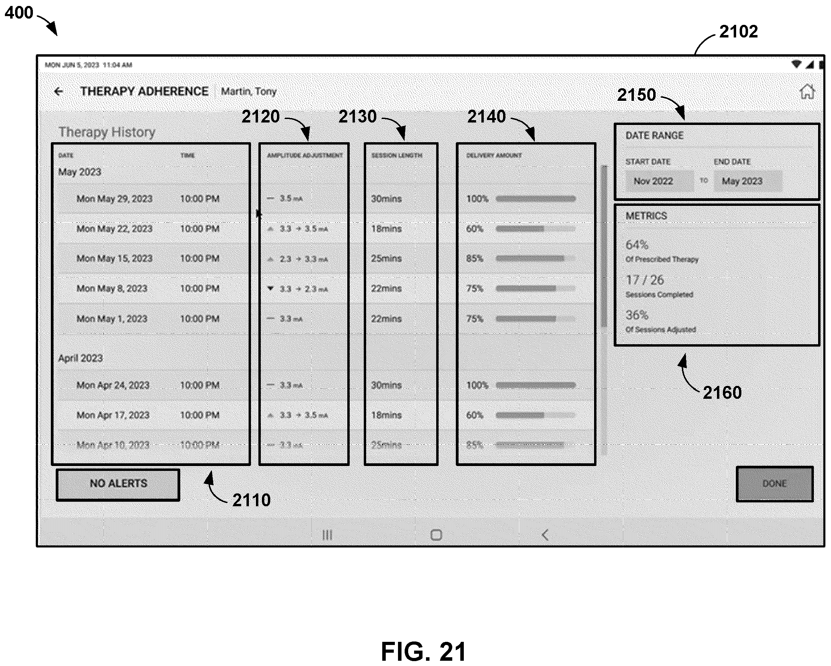

FIG. 21 is a conceptual diagram illustrating an example screen 2102 indicating details and data about a patient's therapy adherence history. Screen 2102 may be produced on user interface 400 when a user clicks the "View Details" button 2018 of screen 2002 as described in relation to FIG. 20. Screen 2102 may have a table displaying various columns of information about a patient's therapy adherence including therapy session date and time column 2110, therapy amplitude adjustment column 2120, session length column 2130, and delivery amount column 2140. Therapy session date and time column 2110 may display the relevant day of the week (Monday, Tuesday, Wednesday, etc.), day of the month (ex. May 27, May 28, etc.), year, and time of day (10:00) that scheduled therapy occurred for a patient. Therapy amplitude adjustment column 2120 may indicate an amount, either positive or negative, that the patient adjusted the therapy amplitude during the therapy session. Session length column 2130 may indicate how long the prescribed therapy session actually, in fact, lasted. Delivery amount column 2140 may indicate a percentage of the programmed therapy duration that was actually completed by the patient. As an illustrative example, delivery amount column 2140 may indicate that a patient completed 100% delivery amount where a patient completed a full 30 minutes of a 30-minute programmed therapy session. As another illustrative example, delivery amount column 2140 may indicate that a patient completed 60% delivery amount where a patient completed 18 minutes of a 30-minute programmed therapy session. Screen 2102 may also provide date filtering box 2150 for a user to select a range of dates to display relevant details of therapy adherence in the table columns of screen 2102 including therapy session date and time column 2110, therapy amplitude adjustment column 2120, session length column 2130, and delivery amount column 2140. Screen 2102 may also include "Metrics" box 2160 which may present such information as percentage of prescribed therapy complete, number of sessions completed as a fraction of the total number of therapy session prescribed or programmed, as well as a percentage of session in which the user adjusted therapy parameters. All information presented on screen 2102 may be printed or otherwise saved allowing a clinician to save for future reference. Therapy data presented on screen 2102 may also be used in connection with other reference information from a patient, including eCAP and accelerometer data.

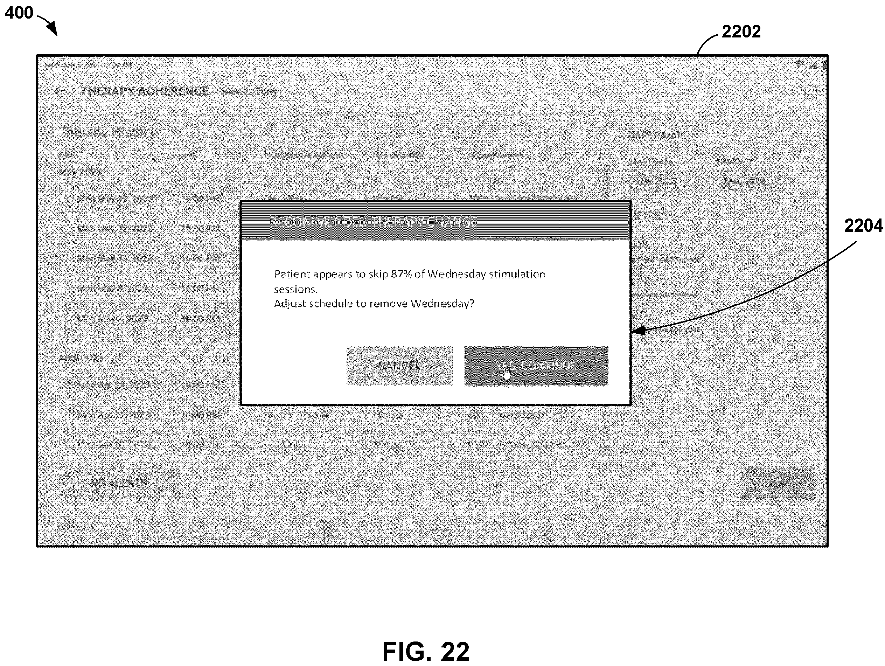

FIG. 22 is a conceptual diagram illustrating an example screen 2202, which includes pop-up window 2204 prompting the user to adjust the patient's therapy schedule based on insights derived from therapy adherence data. Processing circuitry of a programmer, such as processor 202 of programmer 201, or processing circuitry of a server, such as processing circuitry 212 of server 110, may use algorithms configured to recognize patterns from therapy adherence data discussed in connection with screen 2202. For example, an algorithm may be able to recognize that a patient consistently skips therapy sessions of a certain day of the week. In the example of FIG. 22, the algorithm may prompt a notification or screen, such as pop-up window 2204, which prompts a user to adjust the therapy schedule based on the therapy pattern recognized by the algorithm. In some examples, this may include altering a therapy schedule to remove therapy scheduled on a certain day, change the time of therapy on a given day, or change therapy to a different day. In the example of FIG. 22, pop-up window 2204 prompts a user to adjust a therapy schedule by removing or skipping therapy from a certain day of the week (Wednesday), but the algorithm can suggest various changes to the therapy schedule, including adding therapy sessions, shifting therapy sessions, etc. Pop-up window 2204 may also prompt a user to adjust therapy stimulation parameters such as amplitude, pulse width, rate, patient adjustability limits, etc. for individual therapy sessions or a therapy schedule.

In this manner, a system may include processing circuitry (such as processing circuitry 202 of the programmer or processing circuitry 212 of the server 110), which may be configured to receive a first set of information, the first set of information including information of actual therapy delivered to a patient (e.g., information on screen 2102) over a plurality of instances of therapy delivery and determine, based upon the first set of information (i.e., the information of actual therapy delivered to the patient over the plurality of instances of the therapy delivery), a therapy usage pattern. In addition, processing circuitry may be configured to determine a modification to a programmed therapy schedule based on the therapy usage pattern and generate for output the modification to the programmed therapy schedule (e.g., pop-up window 2204). To generate for output, processing circuitry may be configured generate for output, on a display (e.g., user interface 206), the modification to the programmed therapy schedule (e.g., pop-up window 2204). The modification to the programmed therapy schedule may include a recommendation to remove a plurality of instances of therapy sessions from the programmed therapy schedule. The modification to the programmed therapy schedule may include a recommendation to add a plurality of instances of therapy sessions to the programmed therapy schedule. The actual therapy delivered to the patient may include therapy delivered to one or more of a sacral nerve or tibial nerve for incontinence therapy.

In some examples, processing circuitry may be further configured to access a second set of information, the second set of information including information of the programmed therapy schedule for the patient (e.g., information in box 1708 of screen 1702, box 1808 of screen 1802, or box 2008 of screen 2002) and compare the second set of information (i.e., information of the programmed therapy schedule) and the first set of information (i.e., information of the actual therapy delivered to the patient over the plurality of instances of the therapy delivery). To determine the therapy usage pattern, processing circuitry may be configured to determine the therapy usage pattern based upon comparison of the information of the programmed therapy schedule and the information of actual therapy delivered to the patient. The first set of information (i.e., information of actual therapy delivered to the patient) may include a record of skipped and/or adjusted therapy sessions. The first set of information (i.e., information of actual therapy delivered to the patient) may include a record of fully delivered, skipped, adjusted, and/or added therapy sessions. The first set of information (i.e., information of actual therapy delivered to the patient) may include information of incontinence events, number of times a patient went to bathroom, or related events. The first set of information (i.e., information of actual therapy delivered to the patient) may include information input by the user (e.g., patient 106) or automatically record by the processing circuitry, such as processor 202 of programmer 201, or processing circuitry 212 of server 110.

Processing circuitry may be configured to communicate with an implantable medical device (IMD, such as IMD 152). Processing circuitry may be configured to generate for output, on a display, a therapy adherence metric indicative of a patient's adherence to the programmed therapy schedule. The therapy adherence metric may include a percentage of scheduled therapy sessions fully completed by the patient (e.g., box 2014 of screen 2002).

To determine the therapy usage pattern, processing circuitry may be configured to determine a pattern of skipped or adjusted therapy sessions. To determine the pattern of skipped or adjusted therapy sessions, processing circuitry may be configured to determine a pattern of times when one or more stimulation therapy parameters were adjusted relative to one or more stimulation therapy parameters of the programmed therapy schedule and/or a pattern of times when therapy sessions of the programmed therapy schedule were skipped. To determine the therapy usage pattern, processing circuitry may be configured to determine a pattern where therapy delivery is more random, or where there is lack of a pattern. The therapy usage pattern may also include information that a patient can provide regarding their experiences during therapy. For instance, the therapy usage pattern may take into account information of incontinence events, number of times a patient went to bathroom, or related events.

In some examples, processing circuitry, such as processor 202 of programmer 201, or processing circuitry 212 of server 110, may be further configured to generate for output, on a display, a plurality of selectable therapy parameter control icons (e.g., icons of screens 502 or 1002). Processing circuitry, such as processor 202 of programmer 201, or processing circuitry 212 of server 110, may be configured to generate for output, on the display, a plurality of selectable therapy schedule customization icons (e.g., icons of screens 1102, 1302, or 1502). Responsive to receiving input via one or more of the plurality of selectable therapy control icons and via one or more of the plurality of selectable therapy schedule customization icons, processing circuitry, such as processor 202 of programmer 201, or processing circuitry 212 of server 110, may be configured to control an implantable medical device (IMD, e.g. IMD 152) to deliver scheduled therapy. The plurality of selectable therapy parameter control icons may include a plurality of amplitude adjustment icons (e.g., buttons of amplitude selection menu 510). The plurality of selectable therapy parameter control icons may include a plurality of amplitude adjustment icons (e.g., buttons of amplitude selection menu 1010), a plurality of pulse width adjustment icons (e.g., buttons of pulse width selection menu 1020), and a plurality of pulse rate adjustment icons (e.g., buttons of pulse rate selection menu 1030). The plurality of selectable therapy parameter control icons may include a plurality of patient control limit icons (e.g., buttons of "Patient Limit" menu 1050), wherein responsive to receiving input from selection of the plurality of patient control limit icons, the processing circuitry limits therapy schedule adjustments by a patient.

The processing circuitry of one or more devices may be configured in the ways discussed above. A programmer (e.g., programmer 201) may include the processing circuitry, as discussed above. The processing circuitry of one or more devices may be used instead of, or along with, processing circuitry of a programmer. In some examples, one or more servers (e.g., server 110) includes the processing circuitry (e.g., processing circuitry 212). In some examples, the system includes a programmer and one or more servers (e.g., server 110), wherein the processing circuitry includes a first processing circuitry and a second processing circuitry. The programmer (e.g., programmer 201) may include the first processing circuitry (e.g., processing circuitry 202), and the one or more servers (e.g., server 110) may include the second processing circuitry (e.g., processing circuitry 212). The first processing circuitry (e.g., processor 202) may be configured to receive the first set of information (i.e., the information of the actual therapy delivered to the patient over the plurality of instances of therapy delivery). The second processing circuitry (e.g., processing circuitry 212) may be configured to determine, based upon the first set of information (i.e., the information of actual therapy delivered to the patient over the plurality of instances of the therapy delivery), the therapy usage pattern. The second processing circuitry (e.g., processing circuitry 212) may be configured to determine the modification to the programmed therapy schedule based on the therapy usage pattern, The first processing circuitry (e.g., processor 202) may be configured to generate for output the modification to the programmed therapy schedule.

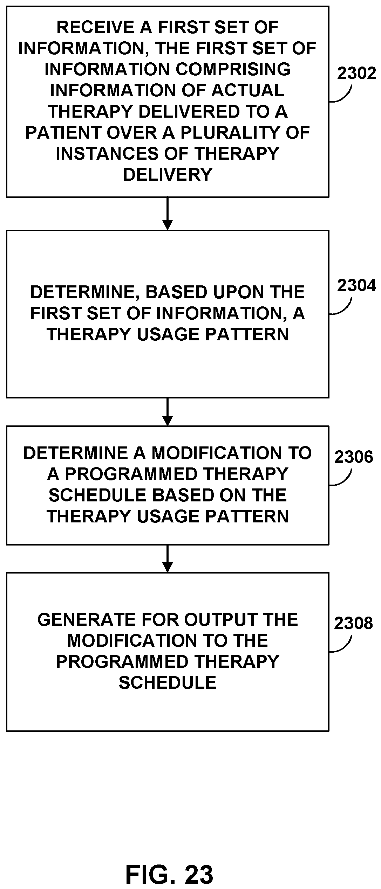

FIG. 23 is a flowchart illustrating an example technique for the programmer calculating diagnostic recommendations to a user based on a patient's therapy adherence. The diagnostic recommendations may allow a user to adjust a therapy schedule based on insights of the patient's therapy adherence. Such processes may utilize the devices and systems of FIGS. 1-3 and user interface shown in FIGS. 4-22. The example techniques may are described as being performed by processing circuitry examples of which include any or any combination of processing circuitry 212, 250, or processor 202.

In the example of FIG. 23, processing circuitry, such as processor 202 of programmer 201, or processing circuitry 212 of server 110, receives a first set of information, the first set of information including information of actual therapy delivered to a patient over a plurality of instances of therapy delivery (2302). Processing circuitry such as processor 202 of programmer 201, or processing circuitry 212 of server 110, determines, based upon the first set of information, a therapy usage pattern (2304). In some examples, the information of therapy actually delivered by a patient may include information recorded by an IMD or a programmer paired to an IMD about therapy delivered to a patient. Information of therapy actually delivered may also include information input by the patient or the clinician. Processing circuitry, such as processor 202 of programmer 201, or processing circuitry 212 of server 110, then determines a modification to a programmed therapy schedule based on the therapy usage pattern (2306). Processing circuitry, such as processor 202 of programmer 201, or processing circuitry 212 of server 110, then generates for output the modification to the programmed therapy schedule (2308). Generating for output may include generating for output on a display. In some examples, the output may be generated for display on a programmer, such as programmer 201. The actual therapy delivered to the patient may include therapy delivered to one or more of a sacral nerve or tibial nerve for incontinence therapy.

Continuing with the example of FIG. 23, the technique may optionally include one or more of the following steps. The processing circuitry, such as processor 202 of programmer 201, or processing circuitry 212 of server 110, may access a second set of information, the second set of information including information of the programmed therapy schedule for the patient. In some examples, programmed therapy schedule information may be information input by a user, such as a clinician, regarding a prescribed therapy schedule for a patient. The processing circuitry, such as processor 202 of programmer 201, or processing circuitry 212 of server 110, may compare the second set of information (i.e., information of the programmed therapy schedule) and the first set of information (i.e., information of the actual therapy delivered to the patient over the plurality of instances of the therapy delivery). To determine the therapy usage pattern, the processing circuitry, such as processor 202 of programmer 201, or processing circuitry 212 of server 110, may determine the therapy usage pattern based upon comparison of the second set of information (i.e., information of the programmed therapy schedule) and the first set of information (i.e., information of the actual therapy delivered to the patient over the plurality of instances of the therapy delivery). The first set of information (i.e., information of actual therapy delivered to the patient) may include a record of skipped and/or adjusted therapy sessions. The first set of information (i.e., information of actual therapy delivered to the patient) may include a record of fully delivered, skipped, adjusted, and/or added therapy sessions. The first set of information (i.e., information of actual therapy delivered to the patient) may include information of incontinence events, number of times a patient went to bathroom, or related events. The first set of information (i.e., information of actual therapy delivered to the patient) may include information input by the user (e.g., patient 106) or automatically record by the processing circuitry, such as processor 202 of programmer 201, or processing circuitry 212 of server 110.

The processing circuitry, such as processor 202 of programmer 201, or processing circuitry 212 of server 110, may communicate with an implantable medical device (IMD). The processing circuitry, such as processor 202 of programmer 201, or processing circuitry 212 of server 110, may generate for output, on a display, a therapy adherence metric indicative of a patient's adherence to the programmed therapy schedule. The therapy adherence metric may include a percentage of scheduled therapy sessions fully completed by the patient.

Continuing with the example of FIG. 23, the technique may optionally include one or more of the following steps. The processing circuitry, such as processor 202 of programmer 201, or processing circuitry 212 of server 110, may generate for output, on a display, a plurality of selectable therapy parameter control icons. The processing circuitry, such as processor 202 of programmer 201, or processing circuitry 212 of server 110, may generate for output, on the display, a plurality of selectable therapy schedule customization icons. Responsive to receiving input via one or more of the plurality of selectable therapy control icons and via one or more of the plurality of selectable therapy schedule customization icons, the processing circuitry, such as processor 202 of programmer 201, or processing circuitry 212 of server 110, may control an implantable medical device (IMD, e.g., IMD 152) to deliver scheduled therapy. The plurality of selectable therapy parameter control icons may include a plurality of amplitude adjustment icons. The plurality of selectable therapy parameter control icons may include a plurality of amplitude adjustment icons, a plurality of pulse width adjustment icons, and a plurality of pulse rate adjustment icons. The plurality of selectable therapy parameter control icons may include a plurality of patient control limit icons, wherein responsive to receiving input from selection of the plurality of patient control limit icons, the processing circuitry, such as processor 202 of programmer 201, or processing circuitry 212 of server 110, limits therapy schedule adjustments by a patient.

Continuing with the example of FIG. 23, the recommended modifications can include changes to stimulation parameters as well as scheduling parameters. For example, the modification to the programmed therapy schedule may include a recommendation to remove a plurality of instances of therapy sessions from the programmed therapy schedule. The modification to the programmed therapy schedule may include a recommendation to add a plurality of instances of therapy sessions to the programmed therapy schedule. The recommended modifications may include a recommendation to reschedule therapy sessions to a different time or to a different day.

Various types of therapy patterns may be recognized and reported as a result of analyzing therapy adherence data in conjunction with step 2304. The therapy pattern may include a pattern of skipped or adjusted therapy sessions. The therapy pattern may include a pattern of fully delivered, skipped, adjusted, and/or added therapy sessions. Determining the pattern of skipped or adjusted therapy sessions may include determining a pattern of times when one or more stimulation therapy parameters were adjusted relative to one or more stimulation therapy parameters of the programmed therapy schedule and/or a pattern of times when therapy sessions of the programmed therapy schedule were skipped. Determining the therapy usage pattern may include determining a pattern where therapy delivery is more random, or where there is lack of a pattern.

Therapy patterns may include instances where a patient consistently adjusts one or more stimulation parameters or scheduling parameters. Therapy patterns may also include instances where a patient randomly adjusts one more stimulation parameters or scheduling parameters. For instance, where a patient consistently adjusts stimulation parameters, an example may include where a patient consistently adjusts the amplitude, pulse width, and/or frequency. As another example, a patient may consistently skip, add, reschedule, or otherwise adjust therapy sessions. For example, a patient may skip, add, reschedule, or otherwise adjust therapy sessions on a given day of the week, or while performing a certain kind of activity (driving, participating in a sporting event, sleeping, etc.). In other examples, where a patient adjusts stimulation parameters in an apparent random fashion, a patient may adjust different stimulation parameters different amounts, adjust parameters at different times, or adjust parameters at inconsistent intervals of time.

In instances where a patient consistently adjusts one or more stimulation parameters or scheduling parameters, a therapy pattern may emerge or may be determined where the patient adjusts a common stimulation or scheduling parameter a number "n" instances out of a total number of scheduled therapy sessions "N." The number of adjusted therapy sessions "n" may correlate to individual therapy sessions, days of the week, or another period of time. The processing circuitry may be configured to determine, recognize, and/or report the therapy pattern when "n" reaches a set threshold "t", or where n/N reaches a set threshold "T" (such as in connection with step 2304). The set threshold "t" where the processing circuitry may determine, recognize, and/or report the therapy pattern may be any number (e.g., "t" = 2, 3, 4, etc.). Accordingly, where "n" is greater than or equal to "t", the processing circuitry may determine, recognize, and/or report the therapy pattern. Similarly, the set threshold "T" where the processing circuitry may determine, recognize, and/or report the therapy pattern may be any fraction (e.g., "T" = 2/3, 3/5, 3/50, etc.). Accordingly, where "n/N" is greater than or equal to "T", the processing circuitry may determine, recognize, and/or report the therapy pattern. The processing circuitry may determine, recognize, and/or report the therapy pattern on a rolling basis, during scheduled therapy, after a certain amount of scheduled therapy sessions, or at certain intervals. The processing circuitry may be able to determine patterns within individual therapy session, within the therapy schedule as a whole, or within both individual therapy sessions and the therapy schedule as a whole.

As an illustrative example, processing circuitry, such as processor 202 of programmer 201, or processing circuitry 212 of server 110, may track days of the week that a patient skips therapy to determine a therapy pattern. The processing circuitry, such as processor 202 of programmer 201, or processing circuitry 212 of server 110, may start tracking such skips after any number weeks of therapy according to a certain schedule (e.g., induction or maintenance schedule). At some point in the future, the processing circuitry, such as processor 202 of programmer 201, or processing circuitry 212 of server 110, may recognize that a patient has skipped a number of therapy session on a given day (e.g., a patient has skipped all Wednesday therapy sessions for seven of the last eight Wednesdays). In such an instance, "n" is seven, and "N" is eight. Where "n" is greater than or equal to a set threshold "t" or "n/N" is greater than or equal to a set threshold "T," the processing circuitry, such as processor 202 of programmer 201, or processing circuitry 212 of server 110, may determine a recommended modification to the therapy schedule and generate for output the recommended modification to the therapy schedule. The recommended modification may include a recommendation to remove therapy from a certain day of the week based on the therapy pattern (e.g., the recommendation may include a recommendation to remove Wednesday sessions from the therapy schedule as shown in the example of FIG. 22).

The processing circuitry of one or more devices may be able to execute the technique described in FIG. 23. For example, a programmer may include the processing circuitry. The programmer may be programmer 201, patient programmer 104, or physician programmer 108 discussed previously. One or more servers (e.g., server 110) may include the processing circuitry. In some examples, the processing circuitry includes a first processing circuitry and a second processing circuitry. A programmer (e.g., programmer 201) may include the first processing circuitry (e.g., processor 202), and one or more servers (e.g., server 110) may include the second processing circuitry (e.g., processing circuitry 212). The first processing circuitry (e.g., processor 202) may be configured to receive the first set of information (i.e., information of the actual therapy delivered to the patient over the plurality of instances of therapy delivery). The second processing circuitry (e.g., processing circuitry 212) may be configured to determine, based upon the first set of information (i.e., the information of actual therapy delivered to the patient over the plurality of instances of the therapy delivery), the therapy usage pattern. The second processing circuitry (e.g., processing circuitry 212) may be configured to determine the modification to the programmed therapy schedule based on the therapy usage pattern. The first processing circuitry (e.g., processor 202) may be configured to generate for output the modification to the programmed therapy schedule.

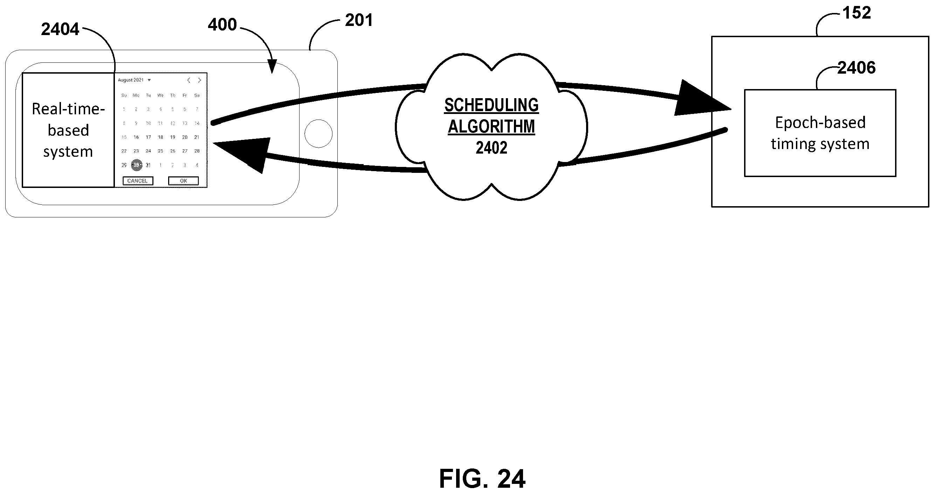

FIG. 24 is a conceptual diagram illustrating communication between a programmer and an implantable medical device via scheduling algorithm 2402. In the example of FIG. 24, the programmer may be programmer 201, but may also be physician programmer 108 or patient programmer 104. Patient programmer may have user interface 400 as discussed in connection with previous figures. Physician programmer 108 may include a real-time-based system 2404 based on real-time clock (which may be a part of processor 202 as discussed in connection with previous figures). By programming therapy using real-time-based system 2404, a user is able to define specific days of a week, month, or year pertaining to a standard Gregorian calendar. Additionally, the implantable medical device (IMD) may be IMD 152 as discussed in connection with previous figures. The IMD may have a different time format compared to the programmer. In some example, IMD 152 employs Epoch-based timing system 2406. Epoch-based timing system 2406 may continually count from a specific programmed Epoch date, such as March 1, 2000. The continuous counting of epoch-based timing system 2406 may in be seconds, or another defined time interval, which continuously counts from the programmed predetermined Epoch date. The advantages of having Epoch-based timing system 2406 in IMD include less memory needed to keep a real-time clock, and in some cases no need to update IMD firmware. The IMD may be able to keep track of time in the Epoch-based timing system 2406 during different battery states in which there is little or no charge. Therefore, the IMD may be able to keep track of time when recovering from low power states, and does not require reprogramming to keep track of therapy schedules.

In the example of FIG. 24, scheduling algorithm 2402 may allow schedules input by the user through physician programmer 108 to be understood by the epoch-based timing system 2406 of IMD 152. For example, scheduling algorithm 2402 may use arithmetic to convert a real-time date of scheduled therapy input of physician programmer 108 to an equivalent amount of time from the Epoch date of epoch-based timing system 2406 such that therapy delivered by IMD 152 matches the therapy scheduled input of programmer 108.

In this manner, a system may include processing circuitry (such as processing circuitry 202 of the programmer or processing circuitry 212 of the server 110), which may be configured to access information of a programmed therapy schedule of a patient. The programmed therapy schedule format may include a real-time-based format. The processing circuitry may be configured to calculate an epoch-based therapy schedule based on the programmed therapy schedule. The epoch-based therapy schedule may include a therapy schedule based on a count relative to a predetermined epoch date. The programmed therapy schedule format may be different than an epoch-based format. The processing circuitry may be configured to deliver, to an implantable medical device having an epoch-based timing system, the epoch-based therapy schedule.

Processing circuitry may be configured to convert the real-time-based format of the programmed therapy schedule to an epoch-based format. Processing circuitry may be configured to receive, from the implantable medical device, the count relative to the predetermined epoch date. Processing circuitry may be configured to receive, from the implantable medical device, an epoch-based record of actual therapy delivered. Processing circuitry may be configured to calculate a real-time-based record of actual therapy delivered. Processing circuitry may be configured to determine a therapy adherence metric from comparison of the real-time-based record of actual therapy delivered and the programmed therapy schedule. The actual therapy delivered to the patient may include therapy delivered to one or more of a sacral nerve or tibial nerve for incontinence therapy.

In some examples, processing circuitry may be configured to generate for output, on a display, a plurality of selectable therapy schedule customization icons. Responsive to receiving input via the plurality of selectable therapy schedule customization icons, processing circuitry may be configured to control the implantable medical device to deliver scheduled therapy. Responsive to receiving input via the plurality of selectable therapy schedule customization icons, processing circuitry may be configured to update the programmed therapy schedule.

The processing circuitry of one or more devices may be configured in the ways discussed above. A programmer (e.g., programmer 201, patient programmer 104, or physician programmer 108) may include the processing circuitry, as discussed above. One or more servers (e.g., server 110) may include the processing circuitry. In some examples, the system may include a programmer and one or more servers, wherein the processing circuitry includes a first processing circuitry and a second processing circuitry. The programmer (e.g., programmer 201) may include the first processing circuitry (e.g., processor 202), and the one or more servers (e.g., server 110) may include the second processing circuitry (e.g., processing circuitry 212). The second processing circuitry (e.g., processing circuitry 212) may be configured to access information of a programmed therapy schedule of the patient. The second processing circuitry (e.g., processing circuitry 212) may be configured to calculate the epoch-based therapy schedule based on the programmed therapy schedule. The first processing circuitry (e.g., processor 202) may be configured deliver, to the implantable medical device (e.g., IMD 152) having the epoch-based timing system, the epoch-based therapy schedule.

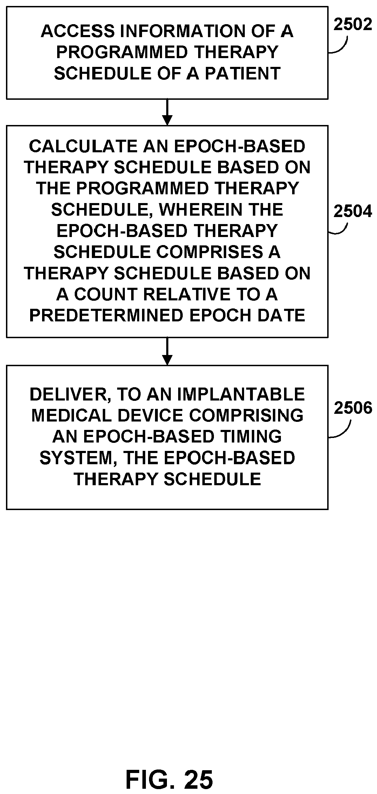

FIG. 25 is a flowchart illustrating an example technique using an algorithm to convert between a real-time based schedule and an Epoch-based timing system of an IMD such that the IMD can delivery therapy according to the real-time schedule. The flow chart of FIG. 25 may use the IMD, user interface, and scheduling algorithm discussed in connection with FIGS. 1-24. In the example of FIG. 25, processing circuitry, such as processor 202 of programmer 201, or processing circuitry 212 of server 110, accesses information of a programmed therapy schedule of a patient (2502). In some examples, programmed therapy schedule information may be information input by a user, such as a clinician, regarding a prescribed therapy schedule for a patient. The programmed therapy schedule format may include a real-time-based format. The processing circuitry, such as processor 202 of programmer 201, or processing circuitry 212 of server 110, calculates an epoch-based therapy schedule based on the programmed therapy schedule, wherein the epoch-based therapy schedule includes a therapy schedule based on a count relative to a predetermined epoch date (2504). In some examples, the programmed therapy schedule format is different than the epoch-based format. The epoch-based therapy schedule may be readable by an epoch-based timing system, such as epoch-based timing system 2406 of IMD (e.g., IMD 152). The scheduling algorithm, such as scheduling algorithm 2402, may convert a real-world date into a count relative to a predetermined epoch date of an epoch-based time system. The processing circuitry, such as processor 202 of programmer 201, or processing circuitry 212 of server 110, delivers to an implantable medical device (e.g., IMD 152) having an epoch-based timing system, the epoch-based therapy schedule (2506). In some examples, the implantable medical device can be IMD 152, which has epoch-based timing system 2406. By receiving the therapy schedule in a format readable by a system having an epoch-based timing system, the IMD is able to delivery therapy that aligns with the real-world therapy schedule.

Continuing with the example of FIG. 25, the technique may optionally include one or more of the following steps. The processing circuitry, such as processor 202 of programmer 201, or processing circuitry 212 of server 110, may convert the real-time-based format of the programmed therapy schedule to an epoch-based format. The processing circuitry, such as processor 202 of programmer 201, or processing circuitry 212 of server 110, may receive, from the implantable medical device, the count relative to the predetermined epoch date. The processing circuitry, such as processor 202 of programmer 201, or processing circuitry 212 of server 110, may receive, from the implantable medical device, an epoch-based record of actual therapy delivered. The processing circuitry, such as processor 202 of programmer 201, or processing circuitry 212 of server 110, may calculate a real-time-based record of actual therapy delivered. The processing circuitry, such as processor 202 of programmer 201, or processing circuitry 212 of server 110, may determine a therapy adherence metric from comparison of the real-time-based record of actual therapy delivered and the programmed therapy schedule. The actual therapy delivered to the patient may include therapy delivered to one or more of a sacral nerve or tibial nerve for incontinence therapy.

In some examples, the processing circuitry, such as processor 202 of programmer 201, or processing circuitry 212 of server 110, may generate on an output a plurality of selectable therapy schedule customization icons. Responsive to receiving input via the plurality of selectable therapy schedule customization icons, the processing circuitry, such as processor 202 of programmer 201, or processing circuitry 212 of server 110, may control the implantable medical device (e.g., IMD 152) to deliver scheduled therapy. Responsive to receiving input via the plurality of selectable therapy schedule customization icons, the processing circuitry, such as processor 202 of programmer 201, or processing circuitry 212 of server 110, may update the programmed therapy schedule.

The processing circuitry of one or more devices may be able to execute the technique described in FIG. 25. For example, a programmer may include the processing circuitry. The programmer may be programmer 201, patient programmer 104, or physician programmer 108 discussed previously. One or more servers (e.g., server 110) may include the processing circuitry (e.g., processing circuitry 212). In some examples, the processing circuitry includes a first processing circuitry and a second processing circuitry. A programmer (e.g., programmer 201) may include the first processing circuitry (e.g., processing circuitry 202), and one or more servers (e.g., server 110) may include the second processing circuitry (e.g., processing circuitry 212). The second processing circuitry (e.g., processing circuitry 212) may be configured to access information of a programmed therapy schedule of the patient. The second processing circuitry (e.g., processing circuitry 212) may be configured to calculate the epoch-based therapy schedule based on the programmed therapy schedule. The first processing circuitry (e.g., processor 202) may be configured deliver, to the implantable medical device (e.g., IMD 152) having the epoch-based timing system, the epoch-based therapy schedule.

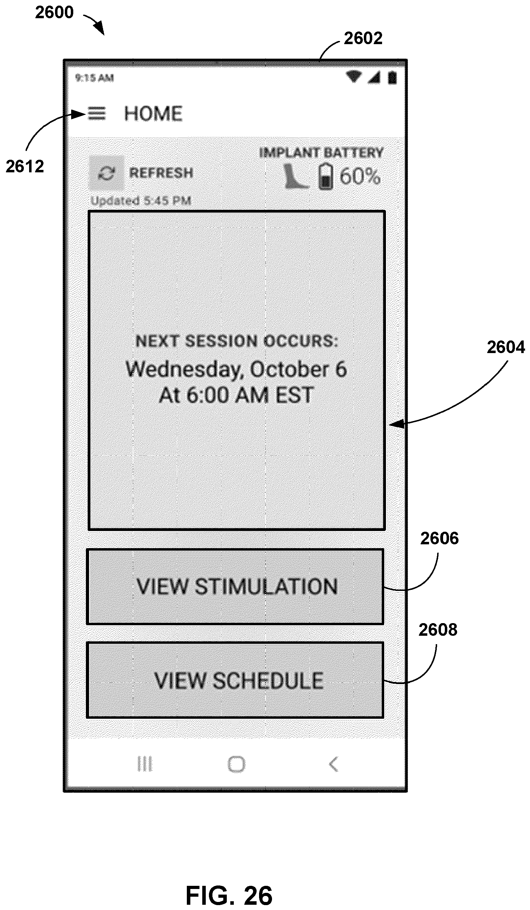

FIGS. 26-31 are conceptual diagrams illustrating example screens of a user interface for a programmer, such as programmer 201, patient programmer 104 or physician programmer 108. FIG. 26 is a conceptual diagram illustrating home screen 2602 when navigating within an example user interface 2600. User interface 2600 may include several different screens as the user can navigate to different functions to view sensed information, view stored data, or adjust various stimulation parameter values and stimulation schedules. In the example of screen 2602, where therapy is not currently being delivered via the IMD, information window 2604 displays information relating to the next scheduled therapy session. Screen 2602 may also have "View Stimulation" button 2606 allowing a user to view more specific information relating to stimulation parameters. Screen 2602 may also have "View Schedule" button 2608 allowing a user to view more specific information relating to stimulation schedules. The programmer may have additional scheduling features which may include the ability to stop a schedule, add "ad-hoc" sessions between scheduled therapy session, skip a therapy session, move scheduled therapy sessions, or otherwise reschedule therapy sessions or therapy schedules. Screen 2602 may have a hamburger menu button 2612, which may open a navigation menu for further options.

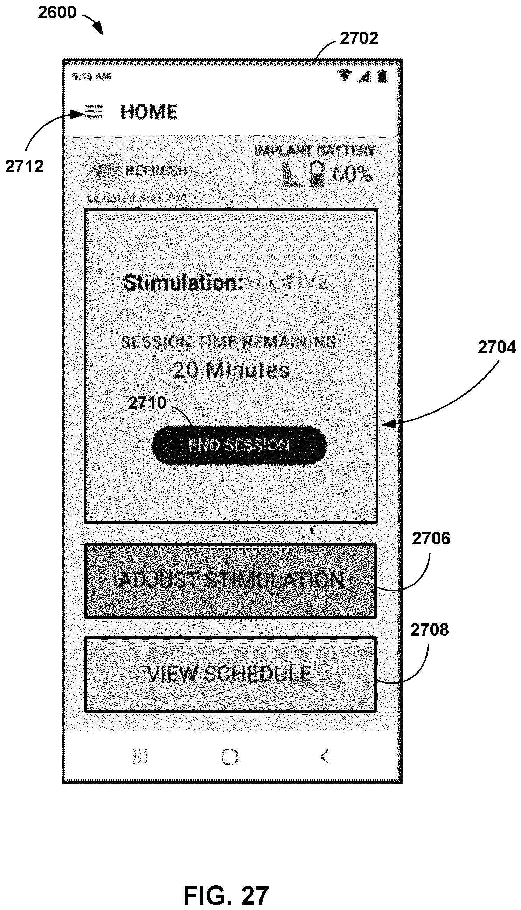

FIG. 27 is a conceptual diagram illustrating an example screen for managing an active therapy session. FIG. 27 is a conceptual diagram illustrating home screen 2702 when navigating within an example user interface 2600. In the example of screen 2702, where therapy is currently being delivered via the IMD, information window 2704 displays information relating to the current active therapy session. For example, information window 2704 may indicate the stimulation status by displaying text such as "Stimulation: ACTIVE" or similar verbiage. Further, information window 2704 may indicate the amount of time remaining in the current session (such as the number of minutes remaining in the therapy session). Information window 2704 may include button 2710, which enables a user to stop the current therapy session when pressed. In the example of information window 2704, button 2710 contains the words "End Session." Screen 2702 may also have "Adjust Stimulation" button 2706 allowing a user to view and/or change more current stimulation parameters. Screen 2702 may also have "View Schedule" button 2708 allowing a user to view more specific information relating to stimulation schedules. Screen 2702 may have a hamburger menu button 2712, which may open a navigation menu for further options.

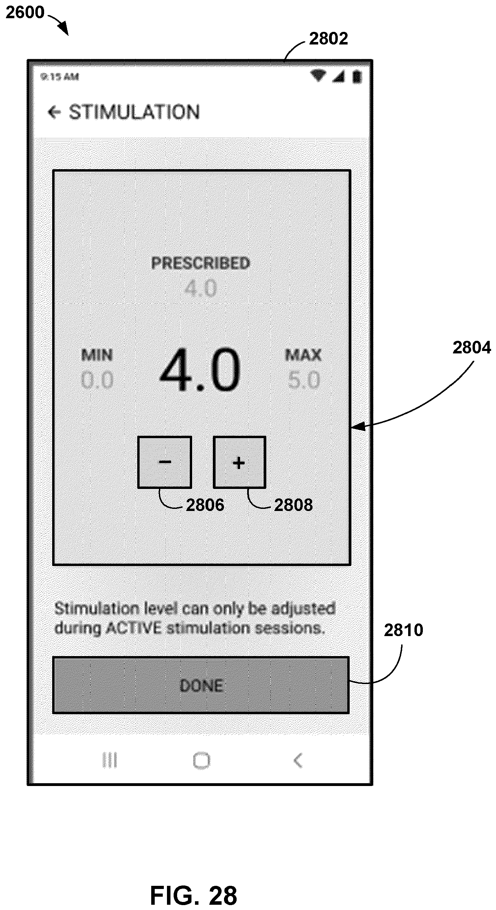

FIG. 28 is a conceptual diagram illustrating an example screen for managing an active therapy session. FIG. 28 includes screen 2802, which is a result of pressing "Adjust Stimulation" button 2706 of screen 2704. Screen 2802 may allow for real-time modification of stimulation parameters during an "Active" therapy session. Screen 2802 may include therapy session parameter information window 2804. Window 2804 may display the current amplitude of therapy (indicated as "4.0" on screen 2802), as well as the "prescribed" target amplitude (also indicated as "4.0" on screen 2802). Window 2804 may also display the minimum and maximum amplitude limits that a patient or user may adjust between. In the example of screen 2802, the minimum and maximum amplitude limits are displayed as "MIN" and "MAX" with corresponding values of "0.0" and "5.0." Window 2804 may contain a plurality of pressable buttons allowing a user to adjust therapy parameters within the minimum and maximum limits during a therapy session. Window 2804 may contain amplitude adjustment button 2806 and button 2808. Button 2806 may enable, when pressed, a user to decrease the amplitude of therapy during a therapy session. Button 2806 may display a minus ("-") sign. Button 2808 may enable, when pressed, a user to increase the amplitude of therapy during a therapy session. Button 2808 may display a plus ("+") sign. Screen 2802 may include a "Done" button 2810, allowing a user to return to previous screens after adjusting therapy parameter levels during an active therapy session.

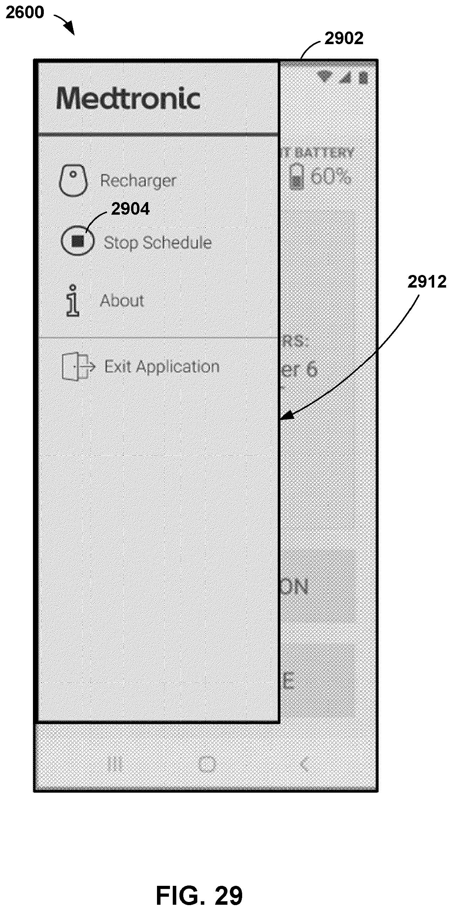

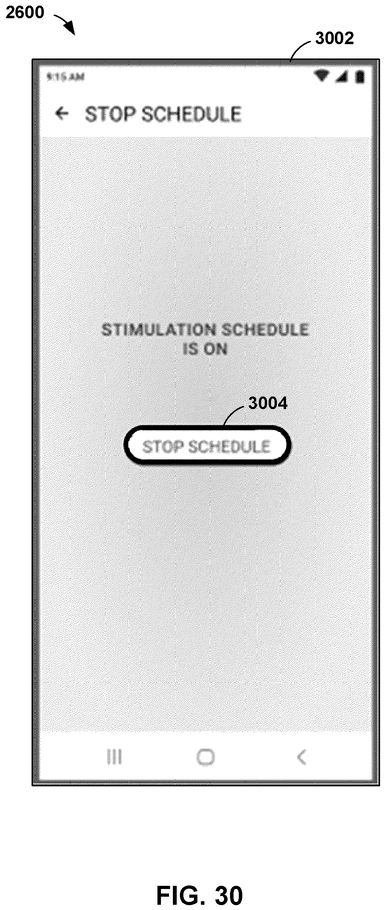

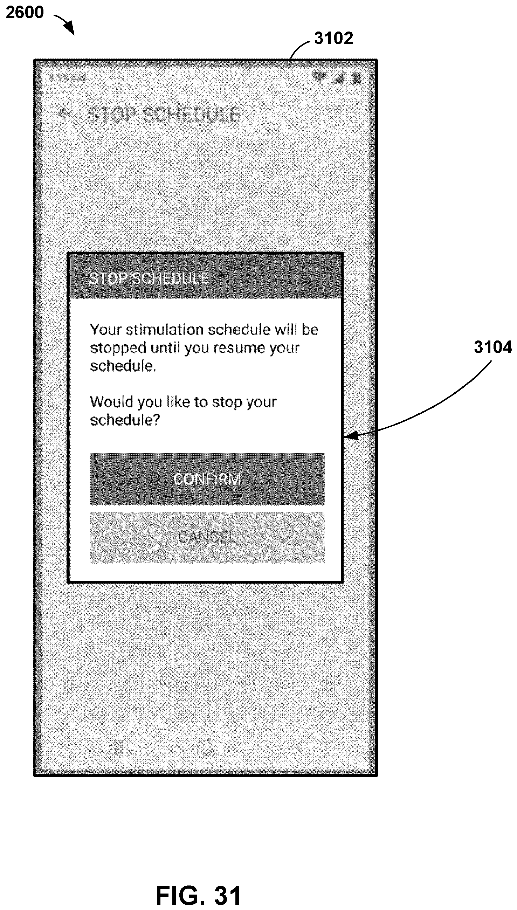

FIG. 29 is a conceptual diagram illustrating an example menu screen for managing a stimulation schedule. FIG. 29 shows an example screen 2902 which includes an example navigation menu 2912. Navigation menu 2912 shows as a result of pressing hamburger menu button 2612 from screen 2602 or hamburger menu button 2712 of screen 2702. Navigation menu 2912 contains "Stop Schedule" button 2904, which, when pressed, begins a series of steps allowing user to stop delivery of future scheduled therapy sessions. FIG. 30 is a conceptual diagram illustrating an example screen for managing a stimulation schedule. FIG. 30 shows example screen 3002, which is presented as a result of pressing "Stop Schedule" button 2904 of screen 2902. Screen 3002 contains "Stop Schedule" button 3004, which, when pressed, may stop the therapy schedule, or may prompt further confirmation of schedule stoppage. FIG. 31 is a conceptual diagram illustrating an example screen 3102, which includes pop-up window 3104 prompting the user to confirm a stoppage of the therapy schedule. Pop-up window 3104 may be the result of pressing the "Stop Schedule" button 3004 of screen 3002. Pop-up window 3104 may have a "Confirm" button allowing a user to confirm stoppage of a therapy schedule and a "Cancel" button to return to a previous screen.

Clause 1. A system comprising: processing circuitry configured to: receive a first set of information, the first set of information comprising information of actual therapy delivered to a patient over a plurality of instances of therapy delivery; determine, based upon the first set of information, a therapy usage pattern; determine a modification to a programmed therapy schedule based on the therapy usage pattern; and generate for output the modification to the programmed therapy schedule.

Clause 2. The system of clause 1, wherein the processing circuitry is further configured to: access a second set of information, the second set of information comprising information of the programmed therapy schedule for the patient; and compare the second set of information and the first set of information, wherein to determine the therapy usage pattern, the processing circuitry is configured to determine the therapy usage pattern based upon comparison of the second set of information and first set of information.

Clause 3. The system of any of clauses 1 and 2, wherein to generate for output, the processing circuitry is configured generate for output, on a display, the modification to the programmed therapy schedule.

Cause 4. The system of any of clauses 1-3, wherein to determine the therapy usage pattern, the processing circuitry is configured to determine a pattern of skipped or adjusted therapy sessions.

Clause 5. The system of clause 4, wherein to determine the pattern of skipped or adjusted therapy sessions, the processing circuitry is configured to determine one or more of: a pattern of times when one or more stimulation therapy parameters were adjusted relative to one or more stimulation therapy parameters of the programmed therapy schedule; and a pattern of times when stimulation sessions of the programmed therapy schedule were skipped.

Clause 6. The system of any of clauses 1-5, wherein the modification to the programmed therapy schedule comprises a recommendation to remove a plurality of instances of therapy sessions from the programmed therapy schedule.

Clause 7. The system of any of clauses 1-6, wherein the modification to the programmed therapy schedule comprises a recommendation to add a plurality of instances of therapy sessions to the programmed therapy schedule.

Clause 8. The system of any of clauses 1-7, wherein the first set of information comprises a record of skipped and/or adjusted therapy sessions.

Clause 9. The system of any of clauses 1-7, wherein the first set of information comprises a record of fully delivered, skipped, adjusted, and/or added therapy sessions.

Clause 10. The system of any of clauses 1-9, wherein the processing circuitry is further configured to communicate with an implantable medical device (IMD).

Clause 11. The system of any of clauses 1-10, wherein the processing circuitry is further configured to: generate for output, on a display, a therapy adherence metric indicative of a patient's adherence to the programmed therapy schedule.

Clause 12. The system of clause 11, wherein the therapy adherence metric comprises a percentage of scheduled therapy sessions fully completed by the patient.

Clause 13. The system of any of clauses 1-12, further comprising a programmer, wherein the programmer comprises the processing circuitry.

Clause 14. The system of any of clauses 1-12, further comprising one or more servers, wherein the one or more servers comprise the processing circuitry.

Clause 15. The system of any of clauses 1-12, further comprising a programmer and one or more servers, wherein the processing circuitry comprises a first processing circuitry and a second processing circuitry, wherein the programmer comprises the first processing circuitry, and the one or more servers comprise the second processing circuitry, and wherein: the first processing circuitry is configured to receive the first set of information, the second processing circuitry is configured to determine, based upon the first set of information, the therapy usage pattern; the second processing circuitry is configured to determine the modification to the programmed therapy schedule based on the therapy usage pattern, and the first processing circuitry is configured to generate for output the modification to the programmed therapy schedule.

Clause 16. The system of any of clauses 1-15, wherein the actual therapy delivered to the patient comprises therapy delivered to one or more of a sacral nerve or tibial nerve for incontinence therapy.

Clause 17. The system of any of clauses 1-16, wherein the processing circuitry is further configured to: generate for output, on a display, a plurality of selectable therapy parameter control icons, generate for output, on the display, a plurality of selectable therapy schedule customization icons, and responsive to receiving input via one or more of the plurality of selectable therapy control icons and via one or more of the plurality of selectable therapy schedule customization icons, control an implantable medical device (IMD) to deliver scheduled therapy.

Clause 18. The system of clause 17, wherein the plurality of selectable therapy parameter control icons comprises a plurality of amplitude adjustment icons.

Clause 19. The system of clause 17, wherein the plurality of selectable therapy parameter control icons comprises a plurality of amplitude adjustment icons, a plurality of pulse width adjustment icons, and a plurality of pulse rate adjustment icons.

Clause 20. The system of any of clauses 17-19, wherein the plurality of selectable therapy parameter control icons comprises a plurality of patient control limit icons, wherein responsive to receiving input from selection of the plurality of patient control limit icons, the processing circuitry limits therapy schedule adjustments by a patient.

Clause 21. A method comprising: receiving, via processing circuitry, a first set of information, the first set of information comprising information of actual therapy delivered to a patient over a plurality of instances of therapy delivery; determining, via processing circuitry, based upon the first set of information, a therapy usage pattern; determining, via processing circuitry, a modification to a programmed therapy schedule based on the therapy usage pattern; and generating for output, via processing circuitry, the modification to the programmed therapy schedule.

Clause 22. The method of clause 21, further comprising: accessing, via processing circuitry, a second set of information, the second set of information comprising information of the programmed therapy schedule for the patient; and comparing, via processing circuitry, the second set of information and the first set of information, wherein determining the therapy usage pattern comprises determining the therapy usage pattern based upon comparison of the second set of information and the first set of information.

Clause 23. The method of any of clauses 21 and 22, wherein generating for output comprises generating for output, on a display, the modification to the programmed therapy schedule.

Clause 24. The method of any of clauses 21-23, wherein determining the therapy usage pattern comprises determining a pattern of skipped or adjusted therapy sessions.

Clause 25. The method of clause 24, wherein determining the pattern of skipped or adjusted therapy sessions comprises determining one or more of: a pattern of times when one or more stimulation therapy parameters were adjusted relative to one or more stimulation therapy parameters of the programmed therapy schedule; and a pattern of times when stimulation sessions of the programmed therapy schedule were skipped.

Clause 26. The method of any of clauses 21-25, wherein the modification to the programmed therapy schedule comprises a recommendation to remove a plurality of instances of therapy sessions from the programmed therapy schedule.

Clause 27. The method of any of clauses 21-26, wherein the modification to the programmed therapy schedule comprises a recommendation to add a plurality of instances of therapy sessions to the programmed therapy schedule.

Clause 28. The method of any of clauses 21-27, wherein the first set of information comprises a record of skipped and/or adjusted therapy sessions.

Clause 29. The method of any of clauses 21-27, wherein the first set of information comprises a record of fully delivered, skipped, adjusted, and/or added therapy sessions.

Clause 30. The method of any of clauses 21-29, further comprising communicating, via the processing circuitry, with an implantable medical device (IMD).

Clause 31. The method of any of clauses 21-30, further comprising:
generating for output, on a display, via the processing circuitry, a therapy adherence metric indicative of a patient's adherence to the programmed therapy schedule.

Clause 32. The method of clause 31, wherein the therapy adherence metric comprises a percentage of scheduled therapy sessions fully completed by the patient.

Clause 33. The method of any of clauses 21-32, wherein a programmer comprises the processing circuitry.

Clause 34. The method of any of clauses 21-32, wherein one or more servers comprise the processing circuitry.

Clause 35. The method of any of clauses 21-32, wherein the processing circuitry comprises a first processing circuitry and a second processing circuitry, wherein a programmer comprises the first processing circuitry, and one or more servers comprise the second processing circuitry, and wherein: the first processing circuitry is configured to receive the first set of information, the second processing circuitry is configured to determine, based upon the first set of information, the therapy usage pattern; the second processing circuitry is configured to determine the modification to the programmed therapy schedule based on the therapy usage pattern; and the first processing circuitry is configured to generate for output the modification to the programmed therapy schedule.

Clause 36. The method of any of clauses 21-35, wherein the actual therapy delivered to the patient comprises therapy delivered to one or more of a sacral nerve or tibial nerve for incontinence therapy.

Clause 37. The method of any of clauses 21-36 further comprising: generating for output, on a display, via the processing circuitry, a plurality of selectable therapy parameter control icons, generating for output, on the display, via the processing circuitry, a plurality of selectable therapy schedule customization icons, and responsive to receiving input via one or more of the plurality of selectable therapy control icons and via one or more of the plurality of selectable therapy schedule customization icons, controlling an implantable medical device (IMD) to deliver scheduled therapy.

Clause 38. The method of clause 37, wherein the plurality of selectable therapy parameter control icons comprises a plurality of amplitude adjustment icons.

Clause 39. The method of clause 37, wherein the plurality of selectable therapy parameter control icons comprises a plurality of amplitude adjustment icons, a plurality of pulse width adjustment icons, and a plurality of pulse rate adjustment icons.

Clause 40. The method of any of clauses 37-39, wherein the plurality of selectable therapy parameter control icons comprises a plurality of patient control limit icons, wherein responsive to receiving input from selection of the plurality of patient control limit icons, the processing circuitry limits therapy schedule adjustments by a patient.

Clause 41. A computer-readable storage medium storing instructions thereon that when executed cause one or more processors to perform the method of any of clauses 21-40.

Clause 42. A system comprising means for performing the method of any of clauses 21-40.

Clause 1A. A system comprising: processing circuitry configured to: access information of a programmed therapy schedule of a patient; calculate an epoch-based therapy schedule based on the programmed therapy schedule, wherein the epoch-based therapy schedule comprises a therapy schedule based on a count relative to a predetermined epoch date; and deliver, to an implantable medical device comprising an epoch-based timing system, the epoch-based therapy schedule.

Clause 2A. The system of clause 1A, wherein a programmed therapy schedule format is different than an epoch-based format.

Clause 3A. The system of clause 1A, wherein a programmed therapy schedule format comprises a real-time-based format.

Clause 4A. The system of clause 3A, wherein the processing circuitry is configured to convert the real-time-based format of the programmed therapy schedule to an epoch-based format.

Clause 5A. The system of any of clauses 1A-4A, wherein the processing circuitry is configured to receive, from the implantable medical device, the count relative to the predetermined epoch date.

Clause 6A. The system of any of clauses 1A-5A, wherein the processing circuitry is further configured to receive, from the implantable medical device, an epoch-based record of actual therapy delivered.

Clause 7A. The system of clause 6A, wherein the processing circuitry is further configured to calculate a real-time-based record of actual therapy delivered.

Clause 8A. The system of clause 7A, wherein the processing circuitry is further configured to determine a therapy adherence metric from comparison of the real-time-based record of actual therapy delivered and the programmed therapy schedule.

Clause 9A. The system of any of clauses 1A-8A, wherein the processing circuitry is further configured to generate for output, on a display, a plurality of selectable therapy schedule customization icons.

Clause 10A. The system of clause 9A, wherein the processing circuitry is further configured to, responsive to receiving input via the plurality of selectable therapy schedule customization icons, control the implantable medical device to deliver scheduled therapy.

Clause 11A. The system of any of clauses 9A-10A, wherein the processing circuitry is further configured to, responsive to receiving input via the plurality of selectable therapy schedule customization icons, update the programmed therapy schedule.

Clause 12A. The system of any of clauses 1A-11A, further comprising a programmer, wherein the programmer comprises the processing circuitry.

Clause 13A. The system of any of clauses 1A-11A, further comprising one or more servers, wherein the one or more servers comprise the processing circuitry.

Clause 14A. The system of any of clauses 1A-11A, further comprising a programmer and one or more servers, wherein the processing circuitry comprises a first processing circuitry and a second processing circuitry, wherein the programmer comprises the first processing circuitry, and the one or more servers comprise the second processing circuitry, and wherein: the second processing circuitry is configured to access information of a programmed therapy schedule of the patient, the second processing circuitry is configured to calculate the epoch-based therapy schedule based on the programmed therapy schedule, and the first processing circuitry is configured deliver, to the implantable medical device comprising the epoch-based timing system, the epoch-based therapy schedule.

Clause 15A. The system of any of clauses 1A-14A, wherein the actual therapy delivered to the patient comprises therapy delivered to one or more of a sacral nerve or tibial nerve for incontinence therapy.

Clause 16A. A method comprising: accessing, via processing circuitry, information of a programmed therapy schedule of a patient; calculating, via processing circuitry, an epoch-based therapy schedule based on the programmed therapy schedule, wherein the epoch-based therapy schedule comprises a therapy schedule based on a count relative to a predetermined epoch date; and delivering, via processing circuitry, to an implantable medical device comprising an epoch-based timing system, the epoch-based therapy schedule.

Clause 17A. The method of clause 16A, wherein a programmed therapy schedule format is different than an epoch-based format.

Clause 18A. The method of clause 16A, wherein a programmed therapy schedule format comprises a real-time-based format.

Clause 19A. The method of clause 18A further comprising converting, via the processing circuitry, the real-time-based format of the programmed therapy schedule to an epoch-based format.

Clause 20A. The method of any of clauses 16A-18A further comprising receiving, from the implantable medical device, the count relative to the predetermined epoch date.

Clause 21A. The method of any of clauses 16A-20A further comprising receiving, from the implantable medical device, an epoch-based record of actual therapy delivered.

Clause 22A. The method of clause 21A further comprising calculating, via the processing circuitry, a real-time-based record of actual therapy delivered.

Clause 23A. The method of clause 22A, further comprising determining, via the processing circuitry, a therapy adherence metric from comparison of the real-time-based record of actual therapy delivered and the programmed therapy schedule.

Clause 24A. The method of any of clauses 16A-23A, further comprising generating for output on a display, via the processing circuitry, a plurality of selectable therapy schedule customization icons.

Clause 25A. The method of clause 24A, further comprising, responsive to receiving input via the plurality of selectable therapy schedule customization icons, controlling the implantable medical device to deliver scheduled therapy.

Clause 26A. The method of any of clauses 24A-25A further comprising, responsive to receiving input via the plurality of selectable therapy schedule customization icons, updating the programmed therapy schedule.

Clause 27A. The method of any of clauses 16A-26A, wherein a programmer comprises the processing circuitry.

Clause 28A. The method of any of clauses 16A-26A, wherein the one or more servers comprise the processing circuitry.

Clause 29A. The method of any of clauses 16A-26A, wherein the processing circuitry comprises a first processing circuitry and a second processing circuitry, wherein a programmer comprises the first processing circuitry, and one or more servers comprise the second processing circuitry, and wherein: the second processing circuitry is configured to access information of a programmed therapy schedule of the patient, the second processing circuitry is configured to calculate the epoch-based therapy schedule based on the programmed therapy schedule, and the first processing circuitry is configured deliver, to the implantable medical device comprising the epoch-based timing system, the epoch-based therapy schedule.

Clause 30A. The method of any of clauses 16A-29A, wherein the actual therapy delivered to the patient comprises therapy delivered to one or more of a sacral nerve or tibial nerve for incontinence therapy.

Clause 31A. A computer-readable storage medium storing instructions thereon that when executed cause one or more processors to perform the method of any of clauses 16A-30A.

Clause 32A. A system comprising means for performing the method of any of clauses 16A-30A.

It will be appreciated by persons skilled in the art that the present application is not limited to what has been particularly shown and described herein above. In addition, unless mention was made above to the contrary, it should be noted that all of the accompanying drawings are not to scale. A variety of modifications and variations are possible in light of the above teachings without departing from the scope and spirit of the application, which is limited only by the following claims.

## Claims

1. A system comprising:
processing circuitry configured to:
receive a first set of information, the first set of information comprising information of actual therapy delivered to a patient over a plurality of instances of therapy delivery;
determine, based upon the first set of information, a therapy usage pattern;
determine a modification to a programmed therapy schedule based on the therapy usage pattern; and
generate for output the modification to the programmed therapy schedule.

2. The system of claim 1, wherein the processing circuitry is further configured to:
access a second set of information, the second set of information comprising information of the programmed therapy schedule for the patient; and
compare the second set of information and the first set of information,
wherein to determine the therapy usage pattern, the processing circuitry is configured to determine the therapy usage pattern based upon comparison of the second set of information and first set of information.

3. The system of any of the preceding claims, wherein to determine the therapy usage pattern, the processing circuitry is configured to determine a pattern of skipped or adjusted therapy sessions.

4. The system of claim 3, wherein to determine the pattern of skipped or adjusted therapy sessions, the processing circuitry is configured to determine one or more of:
a pattern of times when one or more stimulation therapy parameters were adjusted relative to one or more stimulation therapy parameters of the programmed therapy schedule; and
a pattern of times when stimulation sessions of the programmed therapy schedule were skipped.

5. The system of any of the preceding claims, wherein the processing circuitry is further configured to:
generate for output, on a display, a therapy adherence metric indicative of a patient's adherence to the programmed therapy schedule.

6. The system of any of the preceding claims, wherein the actual therapy delivered to the patient comprises therapy delivered to one or more of a sacral nerve or tibial nerve for incontinence therapy.

7. The system of any of the preceding claims, wherein the processing circuitry is further configured to:
generate for output, on a display, a plurality of selectable therapy parameter control icons,
generate for output, on the display, a plurality of selectable therapy schedule customization icons, and
responsive to receiving input via one or more of the plurality of selectable therapy control icons and via one or more of the plurality of selectable therapy schedule customization icons, control an implantable medical device (IMD) to deliver scheduled therapy.

8. The system of claim 7, wherein the plurality of selectable therapy parameter control icons comprises a plurality of amplitude adjustment icons.

9. The system of claim 7, wherein the plurality of selectable therapy parameter control icons comprises a plurality of amplitude adjustment icons, a plurality of pulse width adjustment icons, and a plurality of pulse rate adjustment icons.

10. A method comprising:
receiving, via processing circuitry, a first set of information, the first set of information comprising information of actual therapy delivered to a patient over a plurality of instances of therapy delivery;
determining, via processing circuitry, based upon the first set of information, a therapy usage pattern;
determining, via processing circuitry, a modification to a programmed therapy schedule based on the therapy usage pattern; and
generating for output, via processing circuitry, the modification to the programmed therapy schedule.

11. The method of claim 10, further comprising:
accessing, via processing circuitry, a second set of information, the second set of information comprising information of the programmed therapy schedule for the patient; and
comparing, via processing circuitry, the second set of information and the first set of information,
wherein determining the therapy usage pattern comprises determining the therapy usage pattern based upon comparison of the second set of information and the first set of information.

12. The method of claim 10, wherein determining the therapy usage pattern comprises determining a pattern of skipped or adjusted therapy sessions.

13. The method of claim 12, wherein determining the pattern of skipped or adjusted therapy sessions comprises determining one or more of:
a pattern of times when one or more stimulation therapy parameters were adjusted relative to one or more stimulation therapy parameters of the programmed therapy schedule; and
a pattern of times when stimulation sessions of the programmed therapy schedule were skipped.

14. The method of claim 10, further comprising:
generating for output, on a display, via the processing circuitry, a therapy adherence metric indicative of a patient's adherence to the programmed therapy schedule.

15. A system comprising:
processing circuitry configured to:
access information of a programmed therapy schedule of a patient;
calculate an epoch-based therapy schedule based on the programmed therapy schedule, wherein the epoch-based therapy schedule comprises a therapy schedule based on a count relative to a predetermined epoch date; and
deliver, to an implantable medical device comprising an epoch-based timing system, the epoch-based therapy schedule.
